# EUROPEAN PATENT APPLICATION

(11) **EP 4 344 707 A1**
(43) Date of publication of application: **03.04.2024**
(21) Application number: 22198745.6
(22) Date of filing: 29.09.2022
(51) Int. Cl.: A61K 47/68, A61P 35/00, C07K 16/28

(54) **NEW ANTI-NECTIN-4 ANTIBODY DRUG CONJUGATES**

(71) Applicant: Emergence Therapeutics AG, 47059 Duisburg (DE); Université d'Aix Marseille, 13007 Marseille (FR); INSTITUT NATIONAL DE LA SANTE ET DE LA RECHERCHE MEDICALE - INSERM, 75013 Paris (FR); Institut Jean Paoli & Irène Calmettes, 13009 Marseille (FR); Centre National de la Recherche Scientifique (CNRS), 75016 Paris (FR)
(72) Inventor: ELANDS, Jack, 1190 Forest (BE); LHOSPRICE, Florence, 29770 Primelin (FR); PRÉVILLE, Xavier, 06330 Roquefort les Pins (FR); OLIVE, Daniel, 13009 Marseille (FR); LOPEZ, Marc, 13009 Marseille (FR)
(74) Representative: Weickmann & Weickmann PartmbB

(57) **Abstract**

The present invention relates to an antibody-drug conjugate comprising a monoclonal antibody or an antigen-binding fragment thereof wherein the monoclonal antibody or the antigen-binding fragment thereof binds to Nectin-4 and wherein the drug is a topoisomerase I inhibitor, particularly exatecan or deruxtecan.

## Description

The present invention relates to an antibody-drug conjugate comprising a monoclonal antibody or an antigen-binding fragment thereof wherein the monoclonal antibody or the antigen-binding fragment thereof binds to Nectin-4 and wherein the drug is a topoisomerase I inhibitor, particularly exatecan or deruxtecan.

### BACKGROUND OF THE INVENTION

Nectins are adhesion molecules that help organize epithelial and endothelial junctions and serve as receptors for the entry of the herpes simplex virus, measles virus and poliovirus.

They belong to the immunoglobulin superfamily and are homologs of the poliovirus receptor (PVR / CD155), and for this, have also been called poliovirus receptor bound proteins (PRR). To date, 5 members have been described PVR / CD155, Nectin-1 / PRR1 / CD111, Nectin-2 / PRR2 / CD112, Nectin-3 / PRR3 and Nectin-4 / PRR4. Their ectodomain is made up of three immunoglobulin-like (Ig) -type V, C, C domains that share between 30 and 55% identity in their amino acid sequences.

Expression of Nectin / PRR molecules is generally extensive in tissues, including hematopoietic, neuronal, endothelial and epithelial cells, with the exception of Nectin-3 and -4, which exhibit more restricted expression profiles.

Nectin-4 is a particularly interesting target. It is expressed during fetal development, but its expression decreases and is very restricted in adult tissues in comparison to that of other members of the Nectin family. Nectin-4 is a tumor associated antigen in 83 % of bladder cancers, 78 % of breast cancers (mainly triple negative and ERBB2+), 71 % of pancreatic cancers, 55 % of lung cancers, 57% of ovarian cancers, 59% of head and neck cancers, and 55% of esophageal cancers.

Expression of Nectin-4 in these pathologies is associated with poor prognosis, probably as a consequence of the ability of Nectin-4 to confer to tumor cells *in vitro,* higher capacities of migration, proliferation and formation of metastases. In normal tissues, Nectin-4 is only detected in skin, salivary glands, urinary bladder and esophagus. The recent approval by heath authorities of Enfortumab vedotin for the 2^{nd} line treatment of advanced urothelial cancer has completed the validation of Nectin-4 as a target for the treatment of cancer.

### SUMMARY OF THE INVENTION

A first aspect of the present invention is an antibody-drug conjugate comprising a monoclonal antibody or an antigen-binding fragment thereof wherein the monoclonal antibody or the antigen-binding fragment thereof binds to Nectin-4 and wherein the drug is a topoisomerase I inhibitor, particularly exatecan or deruxtecan.

A further aspect of the present invention is a pharmaceutical composition comprising an active agent, which is an antibody-drug conjugate comprising a monoclonal antibody or an antigen-binding fragment thereof wherein the monoclonal antibody or the antigen-binding fragment thereof binds to Nectin-4 and wherein the drug is a topoisomerase I inhibitor, particularly exatecan, and a pharmaceutically acceptable carrier and/or excipient.

A further aspect of the present invention is the use of an antibody-drug conjugate or a pharmaceutical composition as described above in medicine, particularly in human medicine.

A further aspect of the present invention the use of an antibody-drug conjugate or a pharmaceutical composition as described above in a method for the prevention and/or treatment of a Nectin-4 associated disorder, particularly for the prevention and/or treatment of a Nectin-4 positive cancer.

A further aspect of the present invention the use of an antibody-drug conjugate or a pharmaceutical composition as described above in a method for the prevention and/or treatment of cancer, particularly for the prevention and/or treatment of a cancer selected from bladder, urothelial, endometrial, cervical, colorectal, liver, thyroid, breast, pancreatic, lung, ovarian head and neck and/or esophagus cancer.

A further aspect of the present invention is a method for the prevention and/or treatment of a Nectin-4 associated disorder, particularly for the prevention and/or treatment of a Nectin-4 positive cancer comprising administering to a subject in need thereof a therapeutically effective amount of an antibody-drug conjugate or a pharmaceutical composition as described above.

A further aspect of the present invention is a method for the prevention and/or treatment of cancer, particularly for the prevention and/or treatment of a cancer selected from bladder, urothelial, endometrial, cervical, colorectal, liver, thyroid, breast, pancreatic, lung, ovarian head and neck and/or esophagus cancer comprising administering to a subject in need thereof a therapeutically effective amount of an antibody-drug conjugate or a pharmaceutical composition as described above.

### DETAILED DESCRIPTION OF THE INVENTION

The antibody-drug conjugates of the present invention comprise an anti-Nectin-4 antibody, which specifically binds to Nectin-4 expressed by tumors with a higher affinity in comparison to Nectin-4 expressed by human keratinocytes, e.g., *in vitro* differentiated Nectin-4 expressing keratinocytes. Preferred examples are the monoclonal antibodies (mAb) 15A7.5, 9A2.7, 3A1.4 and/or 8F06.

The present inventors have found that conjugates of anti-Nectin-4 antibodies described herein with topoisomerase-I-inhibitors such as exatecan demonstrate improved anti-cancer activity compared to the marketed antibody Enfortumab vedotin, which is a conjugate of an anti-Nectin-4 antibody and monomethyl auristatin E (MMAE).

An in particular preferred example is monoclonal antibody (mAb) 15A7.5 or an antibody derived therefrom. *In vitro,* this selectivity provides mAb 15A7.5 with lower binding affinity, internalization and/or cytotoxic activity towards keratinocytes in comparison to tumor cells and in reference to the activity of the HA22 mAb (Enfortumab), in the same assays.

In particular, the conjugate of the present invention comprises a humanized antibody that derives from the monoclonal anti-Nectin-4 antibody 15A7.5 mAb. *In vivo,* this low binding capacity to keratinocytes endows mAb 15A7.5 with a higher half-life due to a lower absorption rate in the skin.

In particular the present invention relates to new antibody-drug conjugates comprising such anti-Nectin-4 antibodies, a linker as specified and a topoisomerase-I-inhibitor.

Thus, the present invention relates to an antibody-drug conjugate according to Formula (I) 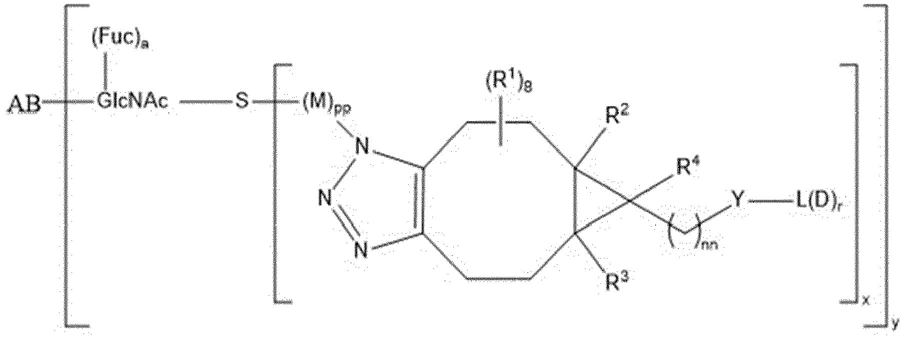 wherein
AB is an anti-Nectin-4 antibody or functional fragment thereof,
Fuc is fucose;
GlcNAc is N-acetylglucosamine;
S is a sugar or sugar derivative;
M is -N(H)C(O)CH₂-, -N(H)C(O)CF₂-, -CH₂-, -CF₂- or a 1,4-phenylene containing 0-4 fluorine substituents, preferably 2 fluorine substituents which are preferably positioned on C2 and C6 or on C3 and C5 of the phenylene;
R¹ is independently selected from the group consisting of hydrogen, halogen, -OR⁸, -NO₂, -CN, S(O)₂R⁸, C₁-C₂₄ alkyl groups, C₆-C₂₄ (hetero)aryl groups, C₇-C₂₄ alkyl(hetero)aryl groups and C₇-C₂₄ (hetero)arylalkyl groups, preferably hydrogen, and wherein the alkyl groups, (hetero)aryl groups, alkyl(hetero)aryl groups and (hetero)arylalkyl groups are optionally substituted, wherein two substituents R⁸ may be linked together to form an annelated cycloalkyl or an annelated (hetero)arene substituent, and wherein R⁸ is independently selected from the group consisting of hydrogen, halogen, C₁-C₂₄ alkyl groups, C₆-C₂₄ (hetero)aryl groups, C₇-C₂₄ alkyl(hetero)aryl groups and C₇-C₂₄ (hetero)arylalkyl groups;
R² and are R³ are independently selected from the group consisting of hydrogen, halogen, C₁-C₂₄ alkyl groups, C₆-C₂₄ (hetero)aryl groups, C₇-C₂₄ alkyl(hetero)aryl groups and C₇-C₂₄ (hetero)arylalkyl groups, preferably hydrogen;
R⁴ is selected from the group consisting of hydrogen, halogen, C₁-C₂₄ alkyl groups, C₆-C₂₄ (hetero)aryl groups, C₇-C₂₄ alkyl(hetero)aryl groups and C₇-C₂₄ (hetero)arylalkyl groups, preferably hydrogen, the alkyl groups optionally being interrupted by one of more hetero-atoms selected from the group consisting of O, N and S, wherein the alkyl groups, (hetero)aryl groups, alkyl(hetero)aryl groups and (hetero)arylalkyl groups are independently optionally substituted;
Y is O, S or NR⁷, wherein R⁷ is independently selected from the group consisting of hydrogen, halogen, -OR⁸, -NO₂, -CN, S(O)₂R⁸, C₁-C₂₄ alkyl groups, C₆-C₂₄ (hetero)aryl groups, C₇-C₂₄ alkyl(hetero)aryl groups and C₇-C₂₄ (hetero)arylalkyl groups and wherein the alkyl groups, (hetero)aryl groups, alkyl(hetero)aryl groups and (hetero)arylalkyl groups are optionally substituted, wherein two substituents R⁸ may be linked together to form an annelated cycloalkyl or an annelated (hetero)arene substituent and wherein R⁸ is independently selected from the group consisting of hydrogen, halogen, C₁-C₂₄ alkyl groups, C₆-C₂₄ (hetero)aryl groups, C₇-C₂₄ alkyl(hetero)aryl groups and C₇-C₂₄ (hetero)arylalkyl groups;
L is a linking group, preferably a sulfamide based linking group,
a is 0 or 1;
x is 1 or 2, preferably 1;
y is 1, 2, 3 or 4, preferably 1,
r is 1, 2, 3, 4, 5, 6, 7, 8, 9, 10, 11, 12, 13, 14, 15, 16, 17, 18, 19 or 20, preferably 1 or 2;
nn is 1, 2, 3, 4, 5, 6, 7, 8, 9 or 10, preferably 1 or 2, in particular 1;
pp is 0 or 1, preferably 1;
D is is a topoisomerase I inhibitor, in particular exatecan.

The general term "sugar" is herein used to indicate a monosaccharide, for example glucose (GIC), galactose (Gal), mannose (Man) and fucose (Fuc).

The term "sugar derivative" is herein used to indicate a derivative of a monosaccharide sugar, i.e. a monosaccharide sugar comprising substituents and/or functional groups.

Examples of a sugar derivative include amino sugars and sugar acids, e.g. glucosamine (GlcNH₂), galactosamine (GalNH₂) N-acetylglucosamine (GlcNAc), N-acetylgalactosamine (GalNAc), sialic acid (Sia) which is also referred to as N-acetylneuraminic acid (NeuNAc), and N-acetylmuramic acid (MurNAc), glucuronic acid (GlcA) and iduronic acid (IdoA). GalNAc is in particular preferred.

GlcNAc moieties as shown in Formula (I) are preferably present at a native N-glycosylation site in the Fc-fragment anti-Nectin-4 antibody or functional fragment thereof. Preferably said GlcNAc moieties are attached to an asparagine amino acid in the region 290-305 of the antibody. In a further preferred embodiment, the antibody is an IgG type antibody, and depending on the particular IgG type antibody, said GlcNAc moieties are present on amino acid asparagine 297 (Asn297 or N297) of the antibody. Of course, other attachment sites, i.e. "attachment amino acids" are also possible.

According to a preferred embodiment b is 1.

According to a preferred embodiment x is 1.

According to a preferred embodiment R¹, R², R³ and R⁴ are hydrogen.

According to a preferred embodiment nn is 1 or 2, in particular 1.

According to a preferred embodiment Y is O. If Y is O, b is preferably 0.

According to a preferred embodiment M is -CH₂-, particular pp being 1.

According a preferred embodiment x is 1, and R¹, R², R³ and R⁴ are hydrogen.

According a preferred embodiment x is 1, and R¹, R², R³ and R⁴ are hydrogen and nn is 1.

According a preferred embodiment x is 1, R¹, R², R³ and R⁴ are hydrogen and nn is 1 and Y is O.

According a preferred embodiment x is 1, R¹, R², R³ and R⁴ are hydrogen, Y is O, nn is 1 and M is -CH₂- with pp being 1.

The compounds disclosed in this description and in the claims may comprise one or more asymmetric centres, and different diastereomers and/or enantiomers may exist of the compounds. The description of any compound in this description and in the claims is meant to include all diastereomers, and mixtures thereof. In addition, the description of any compound in this description and in the claims is meant to include both the individual enantiomers, as well as any mixture of the enantiomers and/or diastereomers. When the structure of a compound is depicted as a specific enantiomer, it is to be understood that the invention of the present application is not limited to that specific enantiomer.

According to a preferred embodiment L comprises a group according to Formula (II) 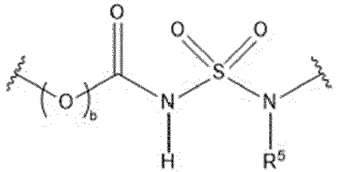 wherein b is 0 or 1;
R⁵ is selected from the group consisting of hydrogen, C₁-C₂₄ alkyl groups, C₃-C₂₄ cycloalkyl groups, C₂-C₂₄ (hetero)aryl groups, C₃-C₂₄ alkyl(hetero)aryl groups and C₃-C₂₄(hetero)arylalkyl groups, preferably hydrogen,
the C₁-C₂₄ alkyl groups, C₃-C₂₄ cycloalkyl groups, C₂-C₂₄ (hetero)aryl groups, C₃-C₂₄ alkyl(hetero)aryl groups and C₃-C₂₄(hetero)arylalkyl groups optionally substituted and/or optionally interrupted by one or more heteroatoms selected from O, S or NR⁶, wherein R⁶ is hydrogen or C₁-C₄ alkyl, preferably hydrogen; wherein D is optionally connected to N via a spacer moiety.

A "spacer moiety" as used herein refers to a moiety that spaces (i.e. provides distance between) and covalently links together two (or more) parts of a linker. The linker may be part of e.g. a linker-construct, the linker-conjugate or a bioconjugate as herein defined.

If Y is O, b is preferably 0.

R⁵ is preferably hydrogen.

According a preferred embodiment x is 1, R¹, R², R³ and R⁴ are hydrogen, Y is O, nn is 1 and M is -CH2- with pp being 1, and L comprises Formula (II), wherein in particular preferred R¹, R², R³, R⁴ and R⁵ are hydrogen.

According to a preferred embodiment the spacer moiety is represented by Formula (III)

Q-(L¹)ₙ-(CO)-(L²)ₒ-(L³)ₚ-(L⁴)_{q}- ,

wherein
- Q: is -(W)ₖ-(A)_{d}-(B)ₑ-(A¹)_{f}- , wherein
W is -OC(O)-, -C(O)O-, -C(O)NH-, -NHC(O)-, -OC(O)NH-,-NHC(O)O-, -C(O)(CH₂)ₘC(O)-, -C(O)(CH₂)ₘC(O)NH-, or-(4-Ph)CH₂NHC(O)(CH₂)ₘC(O)NH-, wherein m is an integer in the range 0-10;
A is of Formula (II) as defined above;
A¹ is of Formula (II) as defined above with R⁵ being preferably (L¹)ₙ-(CO)-(L²)ₒ-(L³)ₚ-(L⁴)_{q}-,
B is a (-CH₂-CH₂-O-)_{g} or a -O-CH₂-CH₂- moiety, or (B)_{g} is a -(CH₂-CH₂-O)_{g}-CH₂CH₂- moiety, wherein g is an integer in the range 1 - 10, preferably being 1 or 2;
k is 0 or 1, with the proviso that if k is 1 then d is 0;
d is 0 or 1;
e is an integer in the range 1 - 10;
f is 0 or 1;
- L¹: is a -CH₂-CH₂-O-, -CH₂-CH₂-O-C(O)-, -O-CH₂-CH₂-, or -(CH₂-CH₂-O)ₙ₁-CH₂-CH₂- moiety, wherein n1 is an independently selected integer in the range of 1-10, preferably -CH₂-CH₂-O- or -CH₂-CH₂-O-C(O)-,
- L²: is a peptide spacer, preferably a dipeptide wherein L² is represented by general structure (IV): 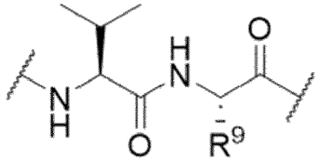 wherein R⁹ is hydrogen, CH₃ or CH₂CH₂CH₂NHC(O)NH₂, preferably R⁹ is hydrogen;
- L³: is self-immolative spacer, preferably a paraaminobenzyloxycarbonyl (PABC) derivative according to structure (V) 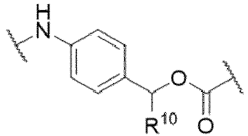
wherein R¹⁰ is hydrogen, R¹¹ or C(O)R¹¹ ,
wherein R¹¹ is selected from C₁-C₂₄ (hetero)alkyl groups, C₃-C₁₀ (hetero)cycloalkyl groups, C₂-C₁₀ (hetero)aryl groups, C₃-C₁₀ alkyl(hetero)aryl groups and C₃-C₁₀ (hetero)arylalkyl groups, which are optionally substituted and optionally interrupted by one or more heteroatoms selected from O, S and NR¹² wherein R¹² is independently selected from the group consisting of hydrogen and C₁-C₄ alkyl groups, R¹⁰ preferably being hydrogen or C(O)R¹¹ wherein R¹¹ is preferably 4-methyl-piperazine or morpholine, most preferably wherein R¹⁰ being hydrogen;
- L⁴: is an aminoalkanoic acid spacer according to the structure -N-(Cₓ-alkylene)-C(O)-, wherein x is an integer in the range 1-10, or L⁴ is an ethyleneglycol spacer according to the structure -N-(CH₂-CH₂-O)ₓ₁-(CH₂)ₓ₂-C(O)-, wherein x1 is an integer in the range 1-10 and x2 is an integer in the range 1-3; and
- n, o, p, q: are independently selected from 0 or 1.

Preferably at least one of d and f is 1.

According to a preferred embodiment R⁵ of A¹ is of-(L¹)ₙ-(CO)-(L²)ₒ-(L³)ₚ-(L⁴)_{q}- as defined herein, i.e. the corresponding N atom is substituted by two residues of Formula

-(L¹)ₙ-(CO)-(L²)ₒ-(L³)ₚ-(L⁴)_{q}-

as defined above. The individual variables may be selected independently. According to a preferred embodiment the residues of Formula -(L¹)ₙ-(CO)-(L²)ₒ-(L³)ₚ-(L⁴)_{q}- are identical.

If Y is O, k is preferably 0, in particular b and k are 0.

"Peptide spacers" as used herein are known by the skilled person preferably comprising 2-5 amino acids, more preferably a dipeptide or tripeptide spacer, most preferably a dipeptide spacer. L² may be selected from Val-Cit, Val-Ala, Val-Lys, Val-Arg Phe-Cit, Phe-Ala, Phe-Lys, Phe-Arg, Ala-Lys, Leu-Cit, Ile-Cit, Trp-Cit, Ala-Ala-Asn, Ala-Asn, more preferably Val-Cit, Val-Ala, Val-Lys, Phe-Cit, Phe-Ala, Phe-Lys, Ala-Ala-Asn, more preferably Val-Cit, Val-Ala, Ala-Ala-Asn. In the particular preferred embodiment the peptide spacer L2 is represented by general formula (IV).

According to a preferred embodiment R⁹ is hydrogen.

According to a preferred embodiment R¹⁰ is hydrogen.

According to a preferred embodiment x is 1, R¹, R², R³ and R⁴ are hydrogen and L² is represented by general structure (IV).

According to a preferred embodiment x is 1, R¹, R², R³ and R⁴ are hydrogen and L² is represented by general structure (IV) and R⁹ is hydrogen.

According to a preferred embodiment x is 1, R¹, R², R³ and R⁴ are hydrogen and L² is represented by general structure (IV), R⁹ is hydrogen and Y is O.

According to a preferred embodiment x is 1, R¹, R², R³ and R⁴ and are hydrogen and L³ is a PABC derivative as herein defined.

According to a preferred embodiment x is 1, R¹, R², R³ and R⁴ and are hydrogen, Y is O, L² is represented by general structure (IV), and L³ is a PABC derivative as herein defined.

According to a preferred embodiment x is 1, R¹, R², R³, R⁴ and R⁹ are hydrogen.

According to a preferred embodiment x is 1, R¹, R², R³, R⁴ and R¹⁰ are hydrogen.

According to a preferred embodiment x is 1, R¹, R², R³, R⁴, R⁹ and R¹⁰ are hydrogen.

According to a preferred embodiment x is 1, R¹, R², R³, R⁴ and R¹⁰ are hydrogen and Y is O.

According to a preferred embodiment x is 1, R¹, R², R³, R⁴ and R¹⁰ are hydrogen, Y is O and L³ is a PABC derivative as herein defined.

According to a preferred embodiment x is 1, R¹, R², R³, R⁴ and R¹⁰ are hydrogen, Y is O, L³ is a PABC derivative as herein defined and M is -CH₂- with pp being 1.

According to a preferred embodiment x is 1, R¹, R², R³, R⁴, R⁹ and R¹⁰ are hydrogen and Y is O.

According an especially preferred embodiment x is 1, R¹, R², R³, R⁴, R⁹ and R¹⁰ are hydrogen, L² is represented by general structure (IV), Y is O and L³ is a PABC derivative as herein defined.

According to an especially preferred embodiment x is 1, R¹, R², R³, R⁴, R⁹ and R¹⁰ are hydrogen, L² is represented by general structure (IV), Y is O, L³ is a PABC derivative as herein defined and M is -CH₂- with pp being 1.

A "self-immolative group" as used herein is a part of a linker in an antibody-drug conjugate with a function to conditionally release a free drug at the site targeted by the ligand unit. The activatable self-immolative moiety may comprise an activatable group (AG) and a self-immolative spacer unit. Upon activation of the activatable group, for example by enzymatic conversion of an amide group to an amino group or by reduction of a disulfide to a free thiol group, a self-immolative reaction sequence is initiated that leads to release of free drug by one or more of various mechanisms. Alternatively, the self-immolative group is not an inherent part of the chemical spacer, but branches off from the chemical spacer connecting the antibody and the payload.

The linker described herein is, for example, described in WO 2021/144313, WO 2016/053107 as well as WO 2017/137456. Methods for attaching the described linkers to antibodies are, for example, described in WO 2011/136645, WO 2022/2022049211, WO 2014/065661, WO 2016/270186, WO 2017/137459 and WO 2021/015622.

### ANTIBODIES

In a particular embodiment, the Nectin-4 binding agent is an anti-Nectin-4 antibody or an antigen-binding fragment thereof.

Typically, the antibody is a monoclonal antibody (mAb) or monoclonal antibody fragment characterized by a specific amino acid sequence. If not indicated differently, the term "monoclonal" refers to a single species, i.e., single amino acid composition of antibodies or antibody fragments.

The antibodies provided herein show preferably specific binding to Nectin-4 and no essential or no cross-reactivity to other proteins of the human Nectin-family, in particular Nectin-1, and/or rodent Nectin-4, such as Nectin-4 from rat and/or mouse.

The antigen-binding site of an inventive antibody comprises heavy chain variable domains/regions (VH) and/or antibody light chain variable domains/regions (VL), or pairs of VH/VL.

The term "antigen-binding site" denotes the region(s) of an antibody molecule to which a ligand (e.g., the antigen, i.e., Nectin-4, or antigen fragment of it) actually binds and which is derived from an antibody.

The variable domains/region denote each of the pair of light and heavy chains, which is involved directly in binding the antibody to the antigen. The variable domain of a heavy chain is abbreviated as "VH" and the variable domain of a light chain is abbreviated as "VL".

An antigen-binding site of an antibody according to the invention can contain six complementarity determining regions (CDRs) which contribute in varying degrees to the affinity of the binding site for the antigen. There are three heavy chain variable domain CDRs (CDR-H1, CDR-H2 and CDR-H3) and three light chain variable domain CDRs (CDR-L1, CDR-L2 and CDR-L3). Also included within the scope of the invention are functional antigen binding sites comprised of fewer CDRs (i.e., where binding specificity is determined by three, four or five CDRs). For example, less than a complete set of 6 CDRs may be sufficient for binding. In some cases, a VH or a VL domain will be sufficient.

According to the present invention, a VH region or the CDRs thereof alone may constitute a complete antigen-binding site. In certain embodiments, the antibody comprises a VH region or the CDRs thereof as defined herein alone. In other embodiments, the antibody comprises a VH region or the CDRs thereof as defined herein together with a VL region or the CDRs thereof, particularly with a VL region or the CDRs thereof as defined herein.

The position of CDRs within a VH or VL region may be defined according to Kabat, et al., Sequences of Proteins of Immunological Interest, 5th Ed. Public Health Service, National Institutes of Health, Bethesda, MD (1991) or the IMGT numbering system, both of which are known to the person skilled in the art. The IMGT numbering has been defined to compare the variable domains whatever the antigen receptor, the chain type, or the species (Lefranc M.-P., "Unique database numbering system for immunogenetic analysis" Immunology Today, 18, 509 (1997); Lefranc M.-P., "The IMGT unique numbering for Immunoglobulins, T cell receptors and Ig-like domains" The Immunologist, 7, 132-136 (1999)). If not stated otherwise, the Kabat system is used herein.

As used herein, the terms "binding" and "specific binding" refer to the binding of the inventive antibody or fragment thereof to an epitope of the Nectin-4 antigen. The measure of the binding strength of an antibody is referred to as affinity. Methods for determining such a binding and/or affinity using in vitro assays are known to the person skilled in the art. According to the present invention, detection with flow cytometry, immuno-histochemistry and/or fluorescence are described and particularly preferred herein.

The affinity of the binding of an antibody to an antigen is defined by the terms Ka (rate constant for the association of the antibody from the antibody/antigen complex), KD (dissociation constant), and K_{dis} (KD/Ka).

In certain embodiments, antibody of the conjugate of the invention may be a chimeric antibody, a multispecific antibody, in particular a bispecific antibody, a human antibody, a humanized antibody, or an antigen-binding fragment thereof.

According to the present invention, a "chimeric" antibody refers to an antibody in which a portion of the heavy and/or light chain is derived from a particular source or species, while the remainder of the heavy and/or light chain is derived from a different source or species.

"Multispecific antibodies" bind two or more different epitopes. The epitopes may be on the same or different antigens. A preferred example of a multispecific antibody is a "bispecific antibody" which binds two different epitopes.

In preferred embodiments, the antibody of the invention is a humanized antibody.

The term "humanized antibody" or "humanized version of an antibody" refers to antibodies for which both heavy and light chains are humanized as a result of antibody engineering. A humanized chain is typically a chain in which the V-region amino acid sequence has been changed so that, analyzed as a whole, is closer in homology to a human germline sequence than to the germline sequence of the species of origin. For example, a murine CDR may be grafted into the framework region of a human antibody to prepare the "humanized antibody." See, e.g., Riechmann, L., et al., Nature 332 (1988) 323-327; and Neuberger, M. S., et al., Nature 314 (1985) 268-270. Other forms of humanized antibodies encompassed by the present invention are those in which the constant region has been additionally modified or changed from that of the original antibody to generate the properties according to the invention. Humanization assessment is based on the resulting amino acid sequence and not on the methodology per se.

Another preferred embodiment refers to conjugates comprising human antibodies.

The term "human antibody", as used herein, is intended to include antibodies having variable and constant regions derived from human germ line immunoglobulin sequences. Human antibodies are well-known in the state of the art (van Dijk, M. A., and van de Winkel, J. G., Curr. Opin. Chem. Biol. 5 (2001) 368-374). Human antibodies can also be produced in transgenic animals (e.g., mice) that are capable, upon immunization, of producing a full repertoire or a selection of human antibodies in the absence of endogenous immunoglobulin production.

The antibody of the conjugate of the present invention may be of any suitable class. The term "class" refers to the type of constant domain or constant region possessed by its heavy chain. As used herein, "constant domain" or "constant region" denotes the sum of the domains of an antibody other than the variable region. The constant region is not directly involved in binding of an antigen but exhibits various effector functions. The antibody may be of any of the five major classes of antibodies, particularly of the five major classes of human antibodies: IgA, IgD, IgE, IgG, and IgM, or any subclass thereof (isotype), e.g., IgG1, IgG2, IgG3, IgG4, IgA1, and IgA2. The heavy chain constant domains that correspond to the different classes of immunoglobulins are called a, δ, ε, γ and µ, respectively. According to the present invention, an antibody of the class, particularly of the human class IgG, IgA or IgM or a fragment thereof is particularly suitable.

According to a preferred embodiment, an antibody of the conjugate of the invention is selected from class IgG, particularly from the class of human IgG, e.g., of subclass IgG1, IgG2, IgG3 of IgG4, of class IgM, of class IgA or an antigen-binding fragment thereof.

In certain embodiments, the antibody comprises a constant domain, particularly a heavy chain constant domain, more particularly a heavy chain constant domain of the class IgG, particularly of the class human IgG, e.g., of subclass IgG1, IgG2, IgG3 of IgG4, of class IgM, of class IgA, which has a reduced effector function compared to a wild-type sequence of the same subclass, e.g., which has a reduced binding to the Fc receptor. Examples of heavy chain constant domains with reduced effector functions may contain at least one of the mutations D265C, L234A, L235A, P331S, L234F and L235E of human IgG, e.g., IgG1 or IgG4 sequences.

An "antigen-binding fragment" of an antibody refers to a molecule comprising a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (e.g., scFv); and multi-specific antibodies formed from antibody fragments. The term also encompasses a fusion protein, e.g., a fusion protein with a non-immunoglobulin peptide or polypeptide, and a conjugate with a non-proteinaceous structure, e.g., a label or a toxin. The terms "antigen-binding fragment of an antibody (thereof)" and "fragment of an antibody (thereof)" may be used interchangeably herein.

The antibody or the antigen-binding fragment may be mono- or multivalent, i.e., it may comprise a single antigen-binding site or multiple antigen-binding sites. For example, Fab fragments have single antigen-binding site, antibodies of the IgG class or Fv or scFv fragments have two antigen-binding sites and antibodies of the IgM class have 5 antigen-binding sites. The term "antibody" also encompasses hetero-specific antibodies, e.g., hetero-bispecific antibodies, which have different antigen-binding sites, particularly antibodies, which are directed to two different epitopes on the antigen. As used herein, "epitope" is a region of an antigen that is bound by an antibody. The term "epitope" includes any polypeptide determinant capable of specific binding to an antibody.

### TUMOR-SELECTIVE NECTIN-4 BINDING AGENTS

In a particular embodiment, the Nectin-4 binding agent of the conjugate is a tumor-selective Nectin-4 binding agent, e.g., a tumor-selective anti-Nectin-4 antibody, which specifically binds to Nectin-4 expressed by tumors with a higher affinity in comparison to Nectin-4 expressed by human keratinocytes, e.g., in vitro differentiated Nectin-4 expressing keratinocytes.

In a further particular embodiment, the conjugate comprises a Nectin-4-binding agent, e.g., an anti-Nectin-4 antibody, which specifically binds to a discontinuous epitope on Nectin-4 consisting of amino acids D57, S58, E60, P133, G135, F137, Q138, and R140 and optionally one or more of G56, G59, E126, R128, P133, and A134 of SEQ ID NO: 112 as determined using Deep Mutational Scanning (DMS) as described in EP 22 186 476.

DMS is known to the person skilled in the art. Basically, every possible amino acid change in a given protein is first synthesized. The activity of each of these protein variants is assayed in parallel using barcodes for each variant. By comparing the activity to the wild-type protein, the effect of each mutation is identified. DMS is, for example, described in detail for example in EP 22 186 476 or by D.M.Fowler and S. Fields inNature Methods 11, 801-807(2014): "Deep mutational scanning: a new style of protein science"*.*

In a further particular embodiment, the tumor-selective Nectin-4 binding agent is a monoclonal antibody derived from the monoclonal antibody (mAb) 15A7.5, 9A2.7, 3A1.4 and/or 8F06. The mAb 15A7.5 is in particular preferred.

For comparison, the binding affinity may be detected by flow cytometry, immuno-histochemistry and/or fluorescence. Such specific binding allows, inter alia, less side effects during a treatment based on the inventive antibodies.

Accordingly, an antibody-drug conjugate comprising antibody 15A7.5, 9A2.7, 3A1.4 and/or 8F06 and in particular a humanized variant derived therefrom, represents a way to improve therapeutic index of Nectin-4 positive cancer treatment through lower associated skin toxicity and higher anti-tumor selectivity and efficacy.

Tumor-selective anti-Nectin-4 antibodies and antigen-binding fragments thereof preferably show a dissociation constant KD of at least 40, preferably at least 45, more preferably at least 50 and most preferably at least 55 nM.

Beside a lower binding affinity, the tumor-selective antibodies and fragments provided herein show also preferably lower internalization and/or cytotoxic activity towards keratinocytes in comparison to tumor cells.

According to an especially preferred embodiment, the tumor-selective antibodies and fragments show lower binding affinity, lower internalization, and lower cytotoxic activity towards keratinocytes in comparison to tumor cells.

Of course, a specific binding to Nectin-4 expressed by tumors is preferred.

In certain embodiments, the tumor-selective monoclonal ant-Nectin-4 antibody or antigen-binding fragment thereof comprises
(a) a variable heavy chain (VH) region comprising complementarity-determining regions (CDRs) CDR-H1, CDR-H2 and CDR-H3, wherein
   (i) the CDR-H1 comprises an amino acid sequence according to SEQ ID NO: 1, 7, 88, 96, or 104,
      or an amino acid sequence comprising a substitution or insertion, particularly a conservative substitution and/or histidine insertion of at least one amino acid, e.g., 1 or 2 amino acids,
   (ii) the CDR-H2 comprises an amino acid sequence according to SEQ ID NO: 2, 8, 89, 97 or 105,
      or an amino acid sequence comprising a substitution or insertion, particularly a conservative substitution and/or histidine insertion at least one amino acid, e.g., 1 or 2 amino acids,
   (iii) the CDR-H3 comprises an amino acid sequence according to SEQ ID NO: 3, 9, 90, 98 or 106,
      or an amino acid sequence comprising a substitution or insertion, particularly a conservative substitution and/or histidine insertion of at least one amino acid, e.g., 1 or 2 amino acids,
      and optionally
(b) a variable light chain (VL) region, particularly a VL region comprising complementarity-determining regions (CDRs) CDR-L1, CDR-L2 and CDR-L3, wherein
   (i) the CDR-L1 comprises an amino acid sequence according to SEQ ID NO: 4, 10, 92, 100 or 108,
      or an amino acid sequence comprising a substitution or insertion, particularly a conservative substitution and/or histidine insertion of at least one amino acid, e.g., 1 or 2 amino acids,
   (ii) the CDR-L2 comprises an amino acid sequence according to SEQ ID NO: 5, 11, 93, 101 or 109,
      or an amino acid sequence comprising a substitution or insertion, particularly a conservative substitution and/or histidine insertion of at least one amino acid, e.g., 1 or 2 amino acids,
   (iii) the CDR-L3 comprises an amino acid sequence according to SEQ ID NO: 6, 12, 94, 102 or 110,
      or an amino acid sequence comprising a substitution or insertion, particularly a conservative substitution and/or histidine insertion of at least one amino acid, e.g., 1 or 2 amino acids.

SEQ ID NO: 1-6 define the six CDR sequences of the antibody 15A7.5 according to the IMGT numbering system. SEQ ID NO: 7-12 define the six CDR sequences of the antibody according to Kabat.

Specific amino acid sequences for the SEQ ID NO: used herein are provided in the attached sequence listing.

SEQ ID NO:s 88-95 characterize mAb 9A2.7.

SEQ ID NO:s 96-103 characterize mAb 3A1.4.

SEQ ID NO:s 104-111 characterize mAb 8F06.

According to the present invention, a substitution or insertion of at least one amino acid, e.g., 1 or 2 amino acids in these CDR sequences is possible. In particular embodiments, a conservative amino acid substitution is preferable, i.e. a substitution of an amino acid by another amino acid with similar biochemical properties, for example a substitution of an aliphatic amino acid, e.g., Gly, Ala, Val, Leu or Ile, for another aliphatic amino acid, a basic amino acid, e.g., His, Lys or Arg, against another basic amino acid, an acidic amino acid or an amide thereof, e.g., Asp, Glu, Asn or Gln, against another acidic amino acid or an amide thereof, an aromatic amino acid, e.g., Phe, Tyr or Trp, against another aromatic amino acid, or a hydroxy or sulfur containing amino acid, e.g., Ser, Thr, Met or Cys, against another hydroxy or sulfur containing amino acid. In further particular embodiments, at least one amino acid, e.g., 1 or 2 histidine residues are inserted into one or more CDR sequences, e.g., CDR1, CDR2 or CDR3 of the heavy chain or the light chain. This might result in a preferential binding at a low pH, e.g., pH between 5.5 to 6.5 versus a neutral pH, e.g., pH 7.0 to 7.5.

According to a further preferred embodiment, the antibody or antigen-binding fragment thereof comprises according to the IMGT numbering system
(i) a VH region comprising the CDR-H1 of SEQ ID NO:1, the CDR-H2 of SEQ ID NO:2, and the CDR-H3 of SEQ ID NO:3,
   and optionally
(ii) a VL region comprising the CDR-L1 of SEQ ID NO:4, the CDR-L2 of the SEQ ID NO:5 and the CDR-L3 of the SEQ ID NO:6.

According to another preferred embodiment, the antibody or antigen-binding fragment thereof comprises according to Kabat
(i) a VH region comprising the CDR-H1 of SEQ ID NO:7, the CDR-H2 of SEQ ID NO:8, and the CDR-H3 of SEQ ID NO:9,
   and optionally
(ii) a VL region comprising the CDR-L1 of SEQ ID NO:10, the CDR-L2 of the SEQ ID NO:11 and the CDR-L3 of the SEQ ID NO:12.

According to another preferred embodiment, the antibody or antigen-binding fragment thereof comprises
(i) a VH region comprising the CDR-H1 of SEQ ID NO:88, the CDR- of SEQ ID NO:89, and the CDR-H3 of SEQ ID NO:90,
   and optionally
(ii) a VL region comprising the CDR-L1 of SEQ ID NO:92, the CDR-L2 of the SEQ ID NO:93 and the CDR-L3 of the SEQ ID NO:94.

According to another preferred embodiment, the antibody or antigen-binding fragment thereof comprises
(i) a VH region comprising the CDR-H1 of SEQ ID NO:96, the CDR- of SEQ ID NO:97, and the CDR-H3 of SEQ ID NO:98,
   and optionally
(ii) a VL region comprising the CDR-L1 of SEQ ID NO:100, the CDR-L2 of the SEQ ID NO:101 and the CDR-L3 of the SEQ ID NO:102.

According to another preferred embodiment, the antibody or antigen-binding fragment thereof comprises
(i) a VH region comprising the CDR-H1 of SEQ ID NO:104, the CDR-H2 of SEQ ID NO:105, and the CDR-H3 of SEQ ID NO:106, and optionally
(ii) a VL region comprising the CDR-L1 of SEQ ID NO:108, the CDR-L2 of the SEQ ID NO:109 and the CDR-L3 of the SEQ ID NO:110.

More particularly, the antibody or antigen-binding fragment thereof may comprise
(a) a VH region comprising an amino acid sequence according to SEQ ID NO:13, 91, 99 or 107 or an amino acid sequence having an identity thereof of at least 85%, at least 90%, at least 95% or at least 99% over the whole length of the sequence, and optionally
(b) a VL region, particularly a VL region comprising an amino acid sequence according to SEQ ID NO:14, 95, 103 or 111 or an amino sequence having an identity thereof of at least 85%, at least 90%, at least 95% or at least 99% over the whole length of the sequence.

According to a preferred embodiment, the antibody or antigen-binding fragment thereof may comprise
(a) a VH region comprising an amino acid sequence according to SEQ ID NO:13 or an amino acid sequence having an identity thereof of at least 85%, at least 90%, at least 95% or at least 99% over the whole length of the sequence,
   and optionally
(b) a VL region, particularly a VL region comprising an amino acid sequence according to SEQ ID NO:14 or an amino sequence having an identity thereof of at least 85%, at least 90%, at least 95% or at least 99% over the whole length of the sequence.

According to a preferred embodiment, the antibody or antigen-binding fragment thereof may comprise
(a) a VH region comprising an amino acid sequence according to SEQ ID NO:91 or an amino acid sequence having an identity thereof of at least 85%, at least 90%, at least 95% or at least 99% over the whole length of the sequence,
   and optionally
(b) a VL region, particularly a VL region comprising an amino acid sequence according to SEQ ID NO:95 or an amino sequence having an identity thereof of at least 85%, at least 90%, at least 95% or at least 99% over the whole length of the sequence.

According to a preferred embodiment, the antibody or antigen-binding fragment thereof may comprise
(a) a VH region comprising an amino acid sequence according to SEQ ID NO:99 or an amino acid sequence having an identity thereof of at least 85%, at least 90%, at least 95% or at least 99% over the whole length of the sequence,
   and optionally
(b) a VL region, particularly a VL region comprising an amino acid sequence according to SEQ ID NO:103 or an amino sequence having an identity thereof of at least 85%, at least 90%, at least 95% or at least 99% over the whole length of the sequence.

According to a preferred embodiment, the antibody or antigen-binding fragment thereof may comprise
(a) a VH region comprising an amino acid sequence according to SEQ ID NO:107 or an amino acid sequence having an identity thereof of at least 85%, at least 90%, at least 95% or at least 99% over the whole length of the sequence,
   and optionally
(b) a VL region, particularly a VL region comprising an amino acid sequence according to SEQ ID NO:111 or an amino sequence having an identity thereof of at least 85%, at least 90%, at least 95% or at least 99% over the whole length of the sequence.

"Percent (%) amino acid sequence identity" with respect to a peptide or polypeptide sequence is defined as the percentage of amino acid residues in a candidate sequence that are identical with the amino acid residues in the specific peptide or polypeptide sequence, after aligning the sequences and introducing gaps, if necessary, to achieve the maximum percent sequence identity, Alignment for purposes of determining percent amino acid sequence identity can be achieved in various ways that are within the skill in the art, for instance, using publicly available computer software such as BLAST.

In particular embodiments, the humanized or human antibodies are defined by combination of at least 3, preferably 6, complementarity-determining regions (CDRs), i.e., relate to antibodies or antigen-binding fragments thereof comprising
(a) a variable heavy chain (VH) region comprising complementarity-determining regions (CDRs) CDR-H1, CDR-H2 and CDR-H3, wherein
   (i) the CDR-H1 comprises an amino acid sequence according to SEQ ID NO:21, 35, 49 or 63,
      or an amino acid sequence comprising a substitution or insertion, particularly a conservative substitution and/or histidine insertion of at least one amino acid, e.g., 1 or 2 amino acids,
   (ii) the CDR-H2 comprises an amino acid sequence according to SEQ ID NO:22, 36, 50 or 64
      or an amino acid sequence comprising a substitution or insertion, particularly a conservative substitution and/or histidine insertion of at least one amino acid, e.g., 1 or 2 amino acids,
   (iii) the CDR-H3 comprises an amino acid sequence according to SEQ ID NO:23, 37, 51 or 65,
      or an amino acid sequence comprising a substitution or insertion, particularly a conservative substitution and/or histidine insertion of at least one amino acid, e.g., 1 or 2 amino acids,
      and optionally
(b) a variable light chain (VL) region, particularly a VL region comprising complementarity-determining regions (CDRs) CDR-L1, CDR-L2 and CDR-L3, wherein
   (i) the CDR-L1 comprises an amino acid sequence according to SEQ ID NO:24, 38, 52 or 66,
      or an amino acid sequence comprising a substitution or insertion, particularly a conservative substitution and/or histidine insertion of at least one amino acid, e.g., 1 or 2 amino acids,
   (ii) the CDR-L2 comprises an amino acid sequence according to SEQ ID NO:25, 39, 53 or 67,
      or an amino acid sequence comprising a substitution or insertion, particularly a conservative substitution and/or histidine insertion of at least one amino acid, e.g., 1 or 2 amino acids,
   (iii) the CDR-L3 comprises an amino acid sequence according to SEQ ID NO:26, 40, 54 or 68,
      or an amino acid sequence comprising a substitution or insertion, particularly a conservative substitution and/or histidine insertion of at least one amino acid, e.g., 1 or 2 amino acids.

Specific humanized or human antibodies may comprise
(a)
   (i) a VH region comprising the CDR-H1 of SEQ ID NO:21, the CDR-H2 of SEQ ID NO:22, and the CDR-H3 of SEQ ID NO:23,
      and optionally
   (ii) a VL region comprising the CDR-L1 of SEQ ID NO:24, the CDR-L2 of the SEQ ID NO:25, and the CDR-L3 of the SEQ ID NO:26, or
(b)
   (i) a VH region comprising the CDR-H1 of SEQ ID NO:35, the CDR-H2 of SEQ ID NO:36, and the CDR-H3 of SEQ ID NO:37,
      and optionally
   (ii) a VL region comprising the CDR-L1 of SEQ ID NO:38, the CDR-L2 of the SEQ ID NO:39, and the CDR-L3 of the SEQ ID NO:40, or
(c)
   (i) a VH region comprising the CDR-H1 of SEQ ID NO:49, the CDR-H2 of SEQ ID NO:50, and the CDR-H3 of SEQ ID NO:51,
      and optionally
   (ii) a VL region comprising the CDR-L1 of SEQ ID NO:52, the CDR-L2 of the SEQ ID NO:53, and the CDR-L3 of the SEQ ID NO:54, or
(d)
   (i) a VH region comprising the CDR-H1 of SEQ ID NO:63, the CDR-H2 of SEQ ID NO:64, and the CDR-H3 of SEQ ID NO:65,
      and optionally
   (ii) a VL region comprising the CDR-L1 of SEQ ID NO:66, the CDR-L2 of the SEQ ID NO:67, and the CDR-L3 of the SEQ ID NO:68, or
(e)
   (i) a VH region comprising the CDR-H1 of SEQ ID NO:21, the CDR-H2 of SEQ ID NO:22, and the CDR-H3 of SEQ ID NO:23,
      and optionally
   (ii) a VL region comprising the CDR-L1 of SEQ ID NO:38, the CDR-L2 of the SEQ ID NO:39, and the CDR-L3 of the SEQ ID NO:40, or
(f)
   (i) a VH region comprising the CDR-H1 of SEQ ID NO:21, the CDR-H2 of SEQ ID NO:22, and the CDR-H3 of SEQ ID NO:23,
      and optionally
   (ii) a VL region comprising the CDR-L1 of SEQ ID NO:52, the CDR-L2 of the SEQ ID NO:5, and the CDR-L3 of the SEQ ID NO:54, or
(g)
   (i) a VH region comprising the CDR-H1 of SEQ ID NO:21, the CDR-H2 of SEQ ID NO:22, and the CDR-H3 of SEQ ID NO:23,
      and optionally
   (ii) a VL region comprising the CDR-L1 of SEQ ID NO:66, the CDR-L2 of the SEQ ID NO:67, and the CDR-L3 of the SEQ ID NO:68, or
(h)
   (i) a VH region comprising the CDR-H1 of SEQ ID NO:35, the CDR-H2 of SEQ ID NO:36, and the CDR-H3 of SEQ ID NO:37,
      and optionally
   (ii) a VL region comprising the CDR-L1 of SEQ ID NO:24, the CDR-L2 of the SEQ ID NO:25, and the CDR-L3 of the SEQ ID NO:26, or
(j)
   (i) a VH region comprising the CDR-H1 of SEQ ID NO:35, the CDR-H2 of SEQ ID NO:36, and the CDR-H3 of SEQ ID NO:37,
      and optionally
   (ii) a VL region comprising the CDR-L1 of SEQ ID NO:52, the CDR-L2 of the SEQ ID NO:53, and the CDR-L3 of the SEQ ID NO:54, or
(k)
   (i) a VH region comprising the CDR-H1 of SEQ ID NO:35, the CDR-H2 of SEQ ID NO:36, and the CDR-H3 of SEQ ID NO:37, and optionally
   (ii) a VL region comprising the CDR-L1 of SEQ ID NO:66, the CDR-L2 of the SEQ ID NO:67, and the CDR-L3 of the SEQ ID NO:68, or
(l)
   (i) a VH region comprising the CDR-H1 of SEQ ID NO:49, the CDR-H2 of SEQ ID NO:50, and the CDR-H3 of SEQ ID NO:51,
      and optionally
   (ii) a VL region comprising the CDR-L1 of SEQ ID NO:24, the CDR-L2 of the SEQ ID NO:25, and the CDR-L3 of the SEQ ID NO:26, or
(m)
   (i) a VH region comprising the CDR-H1 of SEQ ID NO:49, the CDR-H2 of SEQ ID NO:50, and the CDR-H3 of SEQ ID NO:51,
      and optionally
   (ii) a VL region comprising the CDR-L1 of SEQ ID NO:38, the CDR-L2 of the SEQ ID NO:39, and the CDR-L3 of the SEQ ID NO:40, or
(n)
   (i) a VH region comprising the CDR-H1 of SEQ ID NO:49, the CDR-H2 of SEQ ID NO:50, and the CDR-H3 of SEQ ID NO:51,
      and optionally
   (ii) a VL region comprising the CDR-L1 of SEQ ID NO:66, the CDR-L2 of the SEQ ID NO:67, and the CDR-L3 of the SEQ ID NO:68, or
(o)
   (i) a VH region comprising the CDR-H1 of SEQ ID NO:63, the CDR-H2 of SEQ ID NO:64, and the CDR-H3 of SEQ ID NO:65,
      and optionally
   (ii) a VL region comprising the CDR-L1 of SEQ ID NO:24, the CDR-L2 of the SEQ ID NO:25, and the CDR-L3 of the SEQ ID NO:26, or
(p)
   (i) a VH region comprising the CDR-H1 of SEQ ID NO:63, the CDR-H2 of SEQ ID NO:64, and the CDR-H3 of SEQ ID NO:65,
      and optionally
   (ii) a VL region comprising the CDR-L1 of SEQ ID NO:38, the CDR-L2 of the SEQ ID NO:39, and the CDR-L3 of the SEQ ID NO:40, or
(q)
   (i) a VH region comprising the CDR-H1 of SEQ ID NO:63, the CDR-H2 of SEQ ID NO:64, and the CDR-H3 of SEQ ID NO:65,
      and optionally
   (ii) a VL region comprising the CDR-L1 of SEQ ID NO:52, the CDR-L2 of the SEQ ID NO:53, and the CDR-L3 of the SEQ ID NO:54.

In particular preferred are the humanized antibodies (a), (b), (c), (d), (j), (k), (n) and (q) as defined above.

In particular embodiments, the humanized or human antibodies are defined by combination of at least 3, preferably 6, complementarity-determining regions (CDRs), i.e., relate to antibodies or antigen-binding fragments thereof comprising
(a) a variable heavy chain (VH) region comprising complementarity-determining regions (CDRs) CDR-H1, CDR-H2 and CDR-H3, wherein
   (i) the CDR-H1 comprises an amino acid sequence according to SEQ ID NO:21, 35, 49 or 63,
      or an amino acid sequence comprising a substitution or insertion, particularly a conservative substitution and/or histidine insertion of at least one amino acid, e.g., 1 or 2 amino acids,
   (ii) the CDR-H2 comprises an amino acid sequence according to SEQ ID NO:22, 36, 50 or 64
      or an amino acid sequence comprising a substitution or insertion, particularly a conservative substitution and/or histidine insertion of at least one amino acid, e.g., 1 or 2 amino acids,
   (iii) the CDR-H3 comprises an amino acid sequence according to SEQ ID NO:23, 37, 51 or 65,
      or an amino acid sequence comprising a substitution or insertion, particularly a conservative substitution and/or histidine insertion of at least one amino acid, e.g., 1 or 2 amino acids,
      and optionally
(b) a variable light chain (VL) region, particularly a VL region comprising complementarity-determining regions (CDRs) CDR-L1, CDR-L2 and CDR-L3, wherein
   (i) the CDR-L1 comprises an amino acid sequence according to SEQ ID NO:24, 38, 52 or 66,
      or an amino acid sequence comprising a substitution or insertion, particularly a conservative substitution and/or histidine insertion of at least one amino acid, e.g., 1 or 2 amino acids,
   (ii) the CDR-L2 comprises an amino acid sequence according to SEQ ID NO:25, 39, 53 or 67,
      or an amino acid sequence comprising a substitution or insertion, particularly a conservative substitution and/or histidine insertion of at least one amino acid, e.g., 1 or 2 amino acids,
   (iii) the CDR-L3 comprises an amino acid sequence according to SEQ ID NO:26, 40, 54 or 68,
      or an amino acid sequence comprising a substitution or insertion, particularly a conservative substitution and/or histidine insertion of at least one amino acid, e.g., 1 or 2 amino acids.

Specific humanized or human antibodies may comprise
(a)
   (i) a VH region comprising the CDR-H1 of SEQ ID NO:21, the CDR-H2 of SEQ ID NO:22, and the CDR-H3 of SEQ ID NO:23,
      and optionally
   (ii) a VL region comprising the CDR-L1 of SEQ ID NO:24, the CDR-L2 of the SEQ ID NO:25, and the CDR-L3 of the SEQ ID NO:26, or
(b)
   (i) a VH region comprising the CDR-H1 of SEQ ID NO:35, the CDR-H2 of SEQ ID NO:36, and the CDR-H3 of SEQ ID NO:37,
      and optionally
   (ii) a VL region comprising the CDR-L1 of SEQ ID NO:38, the CDR-L2 of the SEQ ID NO:39, and the CDR-L3 of the SEQ ID NO:40, or
(c)
   (i) a VH region comprising the CDR-H1 of SEQ ID NO:49, the CDR-H2 of SEQ ID NO:50, and the CDR-H3 of SEQ ID NO:51,
      and optionally
   (ii) a VL region comprising the CDR-L1 of SEQ ID NO:52, the CDR-L2 of the SEQ ID NO:53, and the CDR-L3 of the SEQ ID NO:54, or
(d)
   (i) a VH region comprising the CDR-H1 of SEQ ID NO:63, the CDR-H2 of SEQ ID NO:64, and the CDR-H3 of SEQ ID NO:65,
      and optionally
   (ii) a VL region comprising the CDR-L1 of SEQ ID NO:66, the CDR-L2 of the SEQ ID NO:67, and the CDR-L3 of the SEQ ID NO:68, or
(e)
   (i) a VH region comprising the CDR-H1 of SEQ ID NO:21, the CDR-H2 of SEQ ID NO:22, and the CDR-H3 of SEQ ID NO:23,
      and optionally
   (ii) a VL region comprising the CDR-L1 of SEQ ID NO:38, the CDR-L2 of the SEQ ID NO:39, and the CDR-L3 of the SEQ ID NO:40, or
(f)
   (i) a VH region comprising the CDR-H1 of SEQ ID NO:21, the CDR-H2 of SEQ ID NO:22, and the CDR-H3 of SEQ ID NO:23,
      and optionally
   (ii) a VL region comprising the CDR-L1 of SEQ ID NO:52, the CDR-L2 of the SEQ ID NO:53, and the CDR-L3 of the SEQ ID NO:54, or
(g)
   (i) a VH region comprising the CDR-H1 of SEQ ID NO:21, the CDR-H2 of SEQ ID NO:22, and the CDR-H3 of SEQ ID NO:23,
      and optionally
   (ii) a VL region comprising the CDR-L1 of SEQ ID NO:66, the CDR-L2 of the SEQ ID NO:67, and the CDR-L3 of the SEQ ID NO:68, or
(h)
   (i) a VH region comprising the CDR-H1 of SEQ ID NO:35, the CDR-H2 of SEQ ID NO:36, and the CDR-H3 of SEQ ID NO:37,
      and optionally
   (ii) a VL region comprising the CDR-L1 of SEQ ID NO:24, the CDR-L2 of the SEQ ID NO:25, and the CDR-L3 of the SEQ ID NO:26, or
(j)
   (i) a VH region comprising the CDR-H1 of SEQ ID NO:35, the CDR-H2 of SEQ ID NO:36, and the CDR-H3 of SEQ ID NO:37,
      and optionally
   (ii) a VL region comprising the CDR-L1 of SEQ ID NO:52, the CDR-L2 of the SEQ ID NO:53, and the CDR-L3 of the SEQ ID NO:54, or
(k)
   (i) a VH region comprising the CDR-H1 of SEQ ID NO:35, the CDR-H2 of SEQ ID NO:36, and the CDR-H3 of SEQ ID NO:37,
      and optionally
   (ii) a VL region comprising the CDR-L1 of SEQ ID NO:66, the CDR-L2 of the SEQ ID NO:67, and the CDR-L3 of the SEQ ID NO:68, or
(l)
   (i) a VH region comprising the CDR-H1 of SEQ ID NO:49, the CDR-H2 of SEQ ID NO:50, and the CDR-H3 of SEQ ID NO:51,
      and optionally
   (ii) a VL region comprising the CDR-L1 of SEQ ID NO:24, the CDR-L2 of the SEQ ID NO:25, and the CDR-L3 of the SEQ ID NO:26, or
(m)
   (i) a VH region comprising the CDR-H1 of SEQ ID NO:49, the CDR-H2 of SEQ ID NO:50, and the CDR-H3 of SEQ ID NO:51,
      and optionally
   (ii) a VL region comprising the CDR-L1 of SEQ ID NO:38, the CDR-L2 of the SEQ ID NO:39, and the CDR-L3 of the SEQ ID NO:40, or
(n)
   (i) a VH region comprising the CDR-H1 of SEQ ID NO:49, the CDR-H2 of SEQ ID NO:50, and the CDR-H3 of SEQ ID NO:51,
      and optionally
   (ii) a VL region comprising the CDR-L1 of SEQ ID NO:66, the CDR-L2 of the SEQ ID NO:67, and the CDR-L3 of the SEQ ID NO:68, or
(o)
   (i) a VH region comprising the CDR-H1 of SEQ ID NO:63, the CDR-H2 of SEQ ID NO:64, and the CDR-H3 of SEQ ID NO:65,
      and optionally
   (ii) a VL region comprising the CDR-L1 of SEQ ID NO:24, the CDR-L2 of the SEQ ID NO:25, and the CDR-L3 of the SEQ ID NO:26, or
(p)
   (i) a VH region comprising the CDR-H1 of SEQ ID NO:63, the CDR-H2 of SEQ ID NO:64, and the CDR-H3 of SEQ ID NO:65,
      and optionally
   (ii) a VL region comprising the CDR-L1 of SEQ ID NO:38, the CDR-L2 of the SEQ ID NO:39, and the CDR-L3 of the SEQ ID NO:40, or
(q)
   (i) a VH region comprising the CDR-H1 of SEQ ID NO:63, the CDR-H2 of SEQ ID NO:64, and the CDR-H3 of SEQ ID NO:65,
      and optionally
   (ii) a VL region comprising the CDR-L1 of SEQ ID NO:52, the CDR-L2 of the SEQ ID NO:53, and the CDR-L3 of the SEQ ID NO:54.

Particularly preferred are the humanized antibodies (a), (b), (c), (d), (j), (k), (n) and (q) as defined above.

Of course, the humanized or human antibodies may be also defined by their VH and/or VL regions.

Such antibodies may comprise
(i) a VH region comprising an amino acid sequence selected from SEQ ID NO:27, 41, 55 or 69 or an amino acid sequence having an identity thereof of at least 85%, at least 90%, at least 95% or at least 99%,
   and optionally
(ii) a VL region, particularly a VL region comprising an amino acid sequence selected from SEQ ID NO:28, 42, 56, 70 or 71 or an amino sequence having an identity thereof of at least 85%, at least 90%, at least 95% or at least 99%.

Specific humanized or human antibodies may be defined by their VH and/or VL regions, wherein such antibodies comprise
(a)
   (i) a VH region comprising an amino acid sequence according to SEQ ID NO:27 or an amino acid sequence having an identity thereof of at least 85%, at least 90%, at least 95% or at least 99%,
      and optionally
   (ii) a VL region, particularly a VL region comprising an amino acid sequence according to SEQ ID NO:28 or an amino sequence having an identity thereof of at least 85%, at least 90%, at least 95% or at least 99%,
      or
(b)
   (i) a VH region comprising an amino acid sequence according to SEQ ID NO:41 or an amino acid sequence having an identity thereof of at least 85%, at least 90%, at least 95% or at least 99%,
      and optionally
   (ii) a VL region, particularly a VL region comprising an amino acid sequence according to SEQ ID NO:42 or an amino sequence having an identity thereof of at least 85%, at least 90%, at least 95% or at least 99%,
      or
(c)
   (i) a VH region comprising an amino acid sequence according to SEQ ID NO:55 or an amino acid sequence having an identity thereof of at least 85%, at least 90%, at least 95% or at least 99%,
      and optionally
   (ii) a VL region, particularly a VL region comprising an amino acid sequence according to SEQ ID NO:56 or an amino sequence having an identity thereof of at least 85%, at least 90%, at least 95% or at least 99%,
      or
(d)
   (i) a VH region comprising an amino acid sequence according to SEQ ID NO:69 or an amino acid sequence having an identity thereof of at least 85%, at least 90%, at least 95% or at least 99%,
      and optionally
   (ii) a VL region, particularly a VL region comprising an amino acid sequence according to SEQ ID NO:70 or 71 or an amino sequence having an identity thereof of at least 85%, at least 90%, at least 95% or at least 99%,
      or
(e)
   (i) a VH region comprising an amino acid sequence according to SEQ ID NO:27 or an amino acid sequence having an identity thereof of at least 85%, at least 90%, at least 95% or at least 99%,
      and optionally
   (ii) a VL region, particularly a VL region comprising an amino acid sequence according to SEQ ID NO:42 or an amino sequence having an identity thereof of at least 85%, at least 90%, at least 95% or at least 99%,
      or
(f)
   (i) a VH region comprising an amino acid sequence according to SEQ ID NO:27 or an amino acid sequence having an identity thereof of at least 85%, at least 90%, at least 95% or at least 99%,
      and optionally
   (ii) a VL region, particularly a VL region comprising an amino acid sequence according to SEQ ID NO:56 or an amino sequence having an identity thereof of at least 85%, at least 90%, at least 95% or at least 99%,
      or
(g)
   (i) a VH region comprising an amino acid sequence according to SEQ ID NO:27 or an amino acid sequence having an identity thereof of at least 85%, at least 90%, at least 95% or at least 99%,
      and optionally
   (ii) a VL region, particularly a VL region comprising an amino acid sequence according to SEQ ID NO:70 or 71 or an amino sequence having an identity thereof of at least 85%, at least 90%, at least 95% or at least 99%,
      or
(h)
   (i) a VH region comprising an amino acid sequence according to SEQ ID NO:41 or an amino acid sequence having an identity thereof of at least 85%, at least 90%, at least 95% or at least 99%,
      and optionally
   (ii) a VL region, particularly a VL region comprising an amino acid sequence according to SEQ ID NO:28 or an amino sequence having an identity thereof of at least 85%, at least 90%, at least 95% or at least 99%,
      or
(j)
   (i) a VH region comprising an amino acid sequence according to SEQ ID NO:41 or an amino acid sequence having an identity thereof of at least 85%, at least 90%, at least 95% or at least 99%,
      and optionally
   (ii) a VL region, particularly a VL region comprising an amino acid sequence according to SEQ ID NO:56 or an amino sequence having an identity thereof of at least 85%, at least 90%, at least 95% or at least 99%,
      or
(k)
   (i) a VH region comprising an amino acid sequence according to SEQ ID NO:41 or an amino acid sequence having an identity thereof of at least 85%, at least 90%, at least 95% or at least 99%,
      and optionally
   (ii) a VL region, particularly a VL region comprising an amino acid sequence according to SEQ ID NO:70 or 71 or an amino sequence having an identity thereof of at least 85%, at least 90%, at least 95% or at least 99%,
      or
(l)
   (i) a VH region comprising an amino acid sequence according to SEQ ID NO:55 or an amino acid sequence having an identity thereof of at least 85%, at least 90%, at least 95% or at least 99%,
      and optionally
   (ii) a VL region, particularly a VL region comprising an amino acid sequence according to SEQ ID NO:28 or an amino sequence having an identity thereof of at least 85%, at least 90%, at least 95% or at least 99%,
      or
(m)
   (i) a VH region comprising an amino acid sequence according to SEQ ID NO:55 or an amino acid sequence having an identity thereof of at least 85%, at least 90%, at least 95% or at least 99%,
      and optionally
   (ii) a VL region, particularly a VL region comprising an amino acid sequence according to SEQ ID NO:42 or an amino sequence having an identity thereof of at least 85%, at least 90%, at least 95% or at least 99%,
      or
(n)
   (i) a VH region comprising an amino acid sequence according to SEQ ID NO:55 or an amino acid sequence having an identity thereof of at least 85%, at least 90%, at least 95% or at least 99%,
      and optionally
   (ii) a VL region, particularly a VL region comprising an amino acid sequence according to SEQ ID NO:70 or 71 or an amino sequence having an identity thereof of at least 85%, at least 90%, at least 95% or at least 99%,
      or
(o)
   (i) a VH region comprising an amino acid sequence according to SEQ ID NO:69 or an amino acid sequence having an identity thereof of at least 85%, at least 90%, at least 95% or at least 99%,
      and optionally
   (ii) a VL region, particularly a VL region comprising an amino acid sequence according to SEQ ID NO:28 or an amino sequence having an identity thereof of at least 85%, at least 90%, at least 95% or at least 99%,
      or
(p)
   (i) a VH region comprising an amino acid sequence according to SEQ ID NO:69 or an amino acid sequence having an identity thereof of at least 85%, at least 90%, at least 95% or at least 99%,
      and optionally
   (ii) a VL region, particularly a VL region comprising an amino acid sequence according to SEQ ID NO:42 or an amino sequence having an identity thereof of at least 85%, at least 90%, at least 95% or at least 99%,
      or
(q)
   (i) a VH region comprising an amino acid sequence according to SEQ ID NO:69 or an amino acid sequence having an identity thereof of at least 85%, at least 90%, at least 95% or at least 99%,
      and optionally
   (ii) a VL region, particularly a VL region comprising an amino acid sequence according to SEQ ID NO:56 or an amino sequence having an identity thereof of at least 85%, at least 90%, at least 95% or at least 99%.

In particular preferred are the humanized antibodies (a), (b), (c), (d), (j), (k), (n) and (q) as defined above.

### FURTHER ANTI-NECTIN-4 ANTIBODIES

In a further embodiment, the antibody of the conjugate is an antibody or an antigen-binding fragment thereof which binds to a discontinuous epitope on Nectin-4 consisting of amino acids L81, H83, Y86, G87, H89, S91, P92 and E95 of SEQ ID NO: 112 as determined using Deep Mutational Scanning (DMS) and as described in EP 22 191 026, the content of which is herein incorporated by reference.

In a further embodiment, the antibody 5A12.2 or an antigen-binding fragment thereof.

SEQ ID NO:s 80-87 characterize mAb 5A12.2.

According to a preferred embodiment, the 5A12.2 antibody or antigen-binding fragment thereof comprises
(i) a VH region comprising the CDR-H1 of SEQ ID NO:80, the CDR- of SEQ ID NO:81, and the CDR-H3 of SEQ ID NO:82,
   and optionally
(ii) a VL region comprising the CDR-L1 of SEQ ID NO:84, the CDR-L2 of the SEQ ID NO:85 and the CDR-L3 of the SEQ ID NO:86.

According to another preferred embodiment, the 5A12.2 antibody or antigen-binding fragment thereof may comprise
(a) a VH region comprising an amino acid sequence according to SEQ ID NO:83 or an amino acid sequence having an identity thereof of at least 85%, at least 90%, at least 95% or at least 99% over the whole length of the sequence,
   and optionally
(b) a VL region, particularly a VL region comprising an amino acid sequence according to SEQ ID NO:87 or an amino sequence having an identity thereof of at least 85%, at least 90%, at least 95% or at least 99% over the whole length of the sequence.

In a further embodiment, the antibody of the conjugate is a humanized derivative of the antibody 5A12.2 or an antigen-binding fragment thereof as described in EP 22 191 026, the content of which is herein incorporated.

In a further embodiment, the antibody of the conjugate is the antibody HA22, which is the antibody of the marketed antibody-drug conjugate Enfortumab vedotin, or a HA22-derived antibody.

HA22 is a human monoclonal anti-Nectin-4 antibody described in WO2012/047724, the content of which is herein incorporated by reference. A HA22 antibody or a HA22-derived antibody particularly refers to an IgG1 antibody comprising a heavy chain G1m17 or G1m3 haplotype sequence optionally with a constant region having a reduced effector function as described above, e.g., the TM mutation: P331S, L234F and L235E, or the LALA mutation: L234A and L235A and a kappa light chain sequence. In particular, the HA22 VH sequence and its human IgG1 constant region is shown in Fig. 3A, and the HA22 VL sequence and its human kappa constant region is shown in Fig. 3B of WO 2012/047724.

In certain embodiments, a HA22-derived monoclonal anti-Nectin-4 antibody or antigen-binding fragment thereof is defined by its CDR sequences and comprises
(a) a variable heavy chain (VH) region
   comprising complementarity-determining regions (CDRs) CDR-H1, CDR-H2 and CDR-H3, wherein
   (i) the CDR-H1 comprises an amino acid sequence according to SEQ ID NO:72,
      or an amino acid sequence comprising a substitution or insertion, particularly a conservative substitution and/or histidine insertion of at least one amino acid, e.g., 1 or 2 amino acids,
   (ii) the CDR-H2 comprises an amino acid sequence according to SEQ ID NO:73,
      or an amino acid sequence comprising a substitution or insertion, particularly a conservative substitution and/or histidine insertion of at least one amino acid, e.g., 1 or 2 amino acids,
   (iii) the CDR-H3 comprises an amino acid sequence according to SEQ ID NO:74,
      or an amino acid sequence comprising a substitution or insertion, particularly a conservative substitution and/or histidine insertion of at least one amino acid, e.g., 1 or 2 amino acids,
      and optionally
(b) a variable light chain (VL) region, particularly a VL region comprising complementarity-determining regions (CDRs) CDR-L1, CDR-L2 and CDR-L3, wherein
   (i) the CDR-L1 comprises an amino acid sequence according to SEQ ID NO:75,
      or an amino acid sequence comprising a substitution or insertion, particularly a conservative substitution and/or histidine insertion of at least one amino acid, e.g., 1 or 2 amino acids,
   (ii) the CDR-L2 comprises an amino acid sequence according to SEQ ID NO:76,
      or an amino acid sequence comprising a substitution or insertion, particularly a conservative substitution and/or histidine insertion of at least one amino acid, e.g., 1 or 2 amino acids,
   (iii) the CDR-L3 comprises an amino acid sequence according to SEQ ID NO:77,
      or an amino acid sequence comprising a substitution or insertion, particularly a conservative substitution and/or histidine insertion of at least one amino acid, e.g., 1 or 2 amino acids.

SEQ ID NO: 72-77 define the six CDR sequences of the parental antibody HA22 according to the IMGT numbering system.

In particular embodiments, a HA22 derived antibody may be defined by its VH and/or VL regions, wherein such antibody comprises
(i) a VH region comprising an amino acid sequence according to SEQ ID NO:78 or an amino acid sequence having an identity thereof of at least 85%, at least 90%, at least 95% or at least 99%,
   and optionally
(ii) a VL region, particularly a VL region comprising an amino acid sequence according to SEQ ID NO:79 or an amino sequence having an identity thereof of at least 85%, at least 90%, at least 95% or at least 99%.

In even more particular embodiments, a HA22 derived antibody comprises
(i) a VH region comprising an amino acid sequence according to SEQ ID NO:78, and
(ii) a VL region comprising an amino acid sequence according to SEQ ID NO:79.

### TOPOISOMERASE I INHIBITORS

The drug of the antibody conjugate of the invention is a topoisomerase I inhibitor. A topoisomerase I inhibitor is a compound, which is capable of forming a ternary complex with topoisomerase I and DNA, thereby preventing DNA re-ligation, and introducing DNA strand breaks in the cellular genome. The topoisomerase I inhibitor may be e.g., selected from camptothecin or analogs thereof, indenoisoquinolines and indolocarbazoles.

In a particular embodiment, the topoisomerase-I-inhibitor is camptothecin or an analog thereof, i.e. a compound comprising the pentacyclic basic structure of camptothecin and modified substituents optionally resulting in the presence of a further ring. Specific examples are camptothecin, topotecan, irinotecan, SN-38, belotecan, exatecan including derivatives thereof such as deruxtecan, lurtotecan or atiratecan.

In a particular embodiment, the topoisomerase I inhibitor is exatecan:

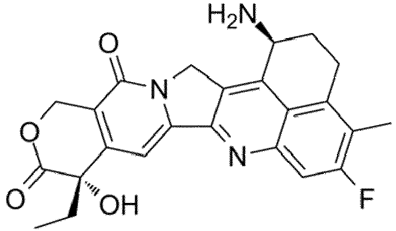

In certain embodiments, exatecan is conjugated to a Nectin-4 binding agent, e.g., an antibody via its NH₂ group.

In further embodiments, the topoisomerase I inhibitor is a camptothecin derivative described in EP 22 177 182.2, the content of which is herein incorporated by reference, represented by the following formula 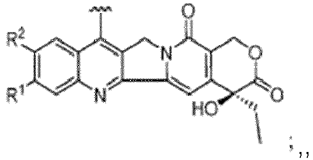 wherein
- R¹: is -F, -CH₃, or -CF₃, preferably -F,
- R²: is -H, -F, -OR³, -SR³, -S(O)R⁴, -S(O)₂R⁴, C₁-C₆ alkyl, or C₁-C₆ fluoroalkyl, preferably -F or methyl; or R¹ and R² taken together with the carbon atoms to which they are attached form a methylene dioxy or a difluoromethylene dioxy ring;
- R³: is H or C₁-C₆ alkyl; and
- R⁴: is C₁-C₆ alkyl.

In still further embodiments, the topoisomerase I inhibitor is a camptothecin derivative described in EP 22 186 381.4, the content of which is herein incorporated by reference, represented by the following formulae 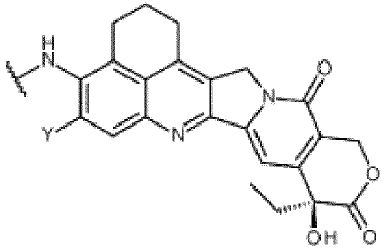 wherein
- Y: is -H, hydroxyl, C₁-C₆ alkyl, C₂-C₆ alkenyl, C₂-C₆ alkynyl, C₁-C₆ alkoxy, C₁-C₆ aminoalkoxy, C₁-C₆ alkylthio, C₁-C₆ hydroxyalkyl, C₁-C₆ haloalkyl, C₁-C₆ cyanoalkyl, C₁-C₆ nitroalkyl, halogen, nitro, cyano, or mercapto, preferably-H or -F,

or 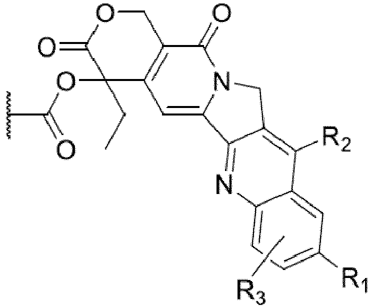 wherein
- R¹: is -CH₃, optionally substituted by halogen, preferably -CF₂H,
- R²: is -H, or C₁-C₆ alkyl, and
- R³: is H, amino, or C₁-C₆ alkyl.

### PHARMACEUTICAL COMPOSITIONS

Further disclosed is a pharmaceutical composition comprising an active agent, which is a conjugate comprising a Nectin-4 binding agent, e.g., a monoclonal antibody or an antigen-binding fragment thereof and a drug which is a topoisomerase I inhibitor, particularly exatecan, and a pharmaceutically acceptable carrier and/or excipient.

Examples of suitable carriers and excipients for formulating antibody drug conjugates include saline and aqueous buffer solutions and are well known in the art. Typically, the pharmaceutical composition is adapted for parenteral administration, e.g., for subcutaneous, intramuscular, or intravenous injection or by infusion. In further embodiments, the pharmaceutical composition is adapted for local administration, e.g., for intravesical instillation into the bladder.

Depending on the stage and the severity of the disorder, the pharmaceutical composition may be administered once or several times in a therapeutically effective dose to a subject in need thereof, particularly to a human subject. For example, it may be administered once or several times daily, each second day, two times weekly or weekly for a suitable period, e.g., of at least one week, or at least one month.

### MEDICAL APPLICATIONS

The conjugate or pharmaceutical composition as described above is used in human medicine for administration to a subject suffering from a drug-resistant Nectin-4 associated disorder.

In certain embodiments, the Nectin-4 associated disorder is a Nectin-4 positive cancer. The cancer may be any kind of cancer, wherein the term "cancer" is used herein to refer to proliferative diseases. Particularly, the cancer is a Nectin-4 positive cancer selected from bladder, urothelial, endometrial, cervical, colorectal, liver, thyroid, breast, e.g., triple negative breast, pancreatic, lung, ovarian, head and neck and/or esophagus cancer.

In particular embodiments, the cancer is a Nectin-4 positive locally advanced or metastatic cancer, e.g., a Nectin-4 positive locally advanced or metastatic urothelial cancer, triple negative breast cancer, cervical cancer or esophagus cancer.

The Nectin-4 associated disorder may be resistant against administration of a conjugate comprising a Nectin-4-binding agent and an auristatin, e.g., MMAE.

In certain embodiments, the disorder is resistant against administration of a conjugate comprising an anti-Nectin-4-antibody and an auristatin. In particular embodiments, the disorder is resistant against administration of a conjugate comprising the anti-Nectin-4-antibody HA22 as described herein above or an antigen-binding fragment thereof.

In certain embodiments, the drug resistant Nectin-4 associated is characterized in that the conjugate comprising a Nectin-4-binding agent and an auristatin has been administered to the subject for a time period of at least 28 days, at least 42 days or at least 56 days.

In certain embodiments, the drug resistant Nectin-4 associated is characterized in that the conjugate comprising a Nectin-4-binding agent and an auristatin, e.g., MMAE, has been administered to the subject as first-line, second-line or third-line treatment.

In certain embodiments, the conjugate comprising a Nectin-4-binding agent and an auristatin, e.g., MMAE, has been administered after an unsuccessful previous treatment. For example, the conjugate comprising a Nectin-4-binding agent and an auristatin has been administered to the subject after a previous treatment comprising administration of a platinum compound such as cisplatin, carboplatin, oxaliplatin or satraplatin, and/or after a previous treatment comprising administration of a checkpoint inhibitor such as nivolumab, pembrolizumab, atezolizumab, or avelumab, and/or after a previous treatment comprising administration of a Mycobacterium bovis cell, particularly Bacille Calmette Guerin (BCG).

In certain embodiments, the resistance of the Nectin-4 associated disorder is characterized by growth of a primary tumor, occurrence and/or reoccurence of tumor metastases, and/or increase of at least one tumor marker.

In therapeutic applications, the active agent is administered in an effective amount to a subject, particularly to a mammalian subject, and more particularly to a human subject. The dose will depend on the specific type of agent, e.g., type of antibody or antibody fragment, the type of disease, and the mode of administration, e.g., locally, or systemically.

In the prevention and/or treatment of cancer, a therapeutic dose of an antibody drug conjugate is typically from about 0.3 mg/kg to about 10 mg/kg.

The antibody drug conjugate may be administered alone or together with a further active agent, which may be selected from chemotherapeutic agents, e.g., anti-metabolites, alkylating agents, intercalating agents, or anti-mitotic agents), inhibitors of specific kinases e.g., tyrosine kinase inhibitors, serine/threonine kinase inhibitors or phosphoinositide kinase inhibitors, immunotherapeutic compounds, e.g., immune checkpoint inhibitors, CAR-T cells, therapeutic vaccines, or oncolytic viruses.

Sequences of SEQ ID. NO. 1 to 112 as disclosed herein are defined as follows:

| | |
|---|---|
| **SEQ ID NO. 1** | **CDR1 VH_15A7.5 IMGT** |
| Gly Phe Thr Phe Ser Asn Tyr Gly | |
| **SEQ ID NO. 2** | **CDR2 VH_15A7.5 IMGT** |
| Ile Ser Asn Leu Ala Tyr Gly Ile | |
| **SEQ ID NO. 3** | **CDR3 VH_15A7.5 IMGT** |
| Ala Arg Gly Ala Arg Ala Thr Gly Trp Phe Ala Tyr | |
| **SEQ ID NO. 4** | **CDR1 VK_15A7.5 IMGT** |
| Gln Asn Val Asp Thr His | |
| **SEQ ID NO. 5** | **CDR2 VK_15A7.5 IMGT** |
| Ser Ala Ser | |
| **SEQ ID NO. 6** | **CDR3 VK_15A7.5 IMGT** |
| Gln Gln Tyr Asn Ser Tyr Pro Leu Thr | |
| **SEQ ID NO. 7** | **CDR1 VH_15A7.5 Kabat** |
| Asn Tyr Gly Met Ala | |
| **SEQ ID NO. 8** | **CDR2 VH_15A7.5 Kabat** |
| Phe Ile Ser Asn Leu Ala Tyr Gly Ile Asn Tyr Ala Asp Thr Val Thr | |
| Gly | |
| **SEQ ID NO. 9** | **CDR3 VH_15A7.5 Kabat** |
| Gly Ala Arg Ala Thr Gly Trp Phe Ala Tyr | |
| **SEQ ID NO. 10** | **CDR1 VK_15A7.5 Kabat** |
| Lys Ala Ser Gln Asn Val Asp Thr His Val Ala | |
| **SEQ ID NO. 11** | **CDR2 VK_15A7.5 Kabat** |
| Ser Ala Ser Tyr Arg Tyr Ser | |
| **SEQ ID NO. 12** | **CDR3 VK_15A7.5 Kabat** |
| Gln Gln Tyr Asn Ser Tyr Pro Leu Thr | |
| **SEQ ID NO. 13** | **VH_15A7.5** |
| Glu Val Lys Leu Val Glu Ser Gly Gly Gly Leu Val Gln Pro Gly Gly Ser Arg Lys Leu Ser Cys Ala Ala Ser Gly Phe Thr Phe Ser Asn Tyr Gly Met Ala Trp Val Arg Gln Ala Pro Gly Lys Gly Pro Glu Trp Val Ala Phe Ile Ser Asn Leu Ala Tyr Gly Ile Asn Tyr Ala Asp Thr Val Thr Gly Arg Phe Thr Ile Ser Arg Glu Asn Ala Lys Asn Thr Leu Tyr Leu Glu Met Arg Ser Leu Arg Ser Glu Asp Thr Ala Met Tyr Tyr Cys Ala Arg Gly Ala Arg Ala Thr Gly Trp Phe Ala Tyr Trp Gly Gln Gly Thr Leu Val Thr Val Ser Ala | |
| **SEQ ID NO.** 14 | **VK_15A7.5** |
| Asp Ile Val Met Thr Gln Ser Gln Lys Phe Met Ser Thr Ser Ile Gly Asp Arg Val Ser Val Thr Cys Lys Ala Ser Gln Asn Val Asp Thr His Val Ala Trp Tyr Gln Glu Lys Pro Gly Gln Ser Pro Lys Ala Leu Ile Tyr Ser Ala Ser Tyr Arg Tyr Ser Gly Val Pro Asp Arg Phe Thr Gly Ser Gly Ser Gly Thr Asp Phe Thr Leu Thr Ile Ser Asn Val Gln Ser Glu Asp Leu Ala Asp Tyr Phe Cys Gln Gln Tyr Asn Ser Tyr Pro Leu Thr Phe Gly Gly Gly Thr Lys Leu Glu Ile Lys | |
| **SEQ ID NO. 15** | **CDR1 H0_15A7.5 IMGT** |
| Gly Phe Thr Phe Ser Asn Tyr Gly Met | |
| **SEQ ID NO. 16** | **CDR2 H0_15A7.5 IMGT** |
| Ile Ser Asn Leu Ala Tyr Gly Ile | |
| **SEQ ID NO. 17** | **CDR3 H0_15A7.5 IMGT** |
| Ala Arg Gly Ala Arg Ala Thr Gly Trp Phe Ala Tyr | |
| **SEQ ID NO. 18** | **CDR1 L0_15A7.5 IMGT** |
| Gln Asn Val Asp Thr His | |
| **SEQ ID NO. 19** | **CDR2 L0_15A7.5 IMGT** |
| Ser Ala Ser | |
| **SEQ ID NO. 20** | **CDR3 L0_15A7.5 IMGT** |
| Gln Gln Tyr Asn Ser Tyr Pro Leu Thr | |
| **SEQ ID NO. 21** | **CDR1 H0_15A7.5 Kabat** |
| Asn Tyr Gly Met Asn | |
| **SEQ ID NO. 22** | **CDR2 H0_15A7.5 Kabat** |
| Phe Ile Ser Asn Leu Ala Tyr Gly Ile Asn Tyr Ala Asp Ser Val Lys | |
| Gly | |
| **SEQ ID NO. 23** | **CDR3 H0_15A7.5 Kabat** |
| Gly Ala Arg Ala Thr Gly Trp Phe Ala Tyr | |
| **SEQ ID NO. 24** | **CDR1 L0_15A7.5 Kabat** |
| Arg Ala Ser Gln Asn Val Asp Thr His Val Ala | |
| **SEQ ID NO. 25** | **CDR2 L0_15A7.5 Kabat** |
| Ser Ala Ser Tyr Leu Gln Ser | |
| **SEQ ID NO. 26** | **CDR3 L0_15A7.5 Kabat** |
| Gln Gln Tyr Asn Ser Tyr Pro Leu Thr | |
| **SEQ ID NO. 27** | **H0_15A7.5** |
| Glu Val Gln Leu Val Glu Ser Gly Gly Gly Leu Val Gln Pro Gly Gly Ser Leu Arg Leu Ser Cys Ala Ala Ser Gly Phe Thr Phe Ser Asn Tyr Gly Met Asn Trp Val Arg Gln Ala Pro Gly Lys Gly Leu Glu Trp Val Ser Phe Ile Ser Asn Leu Ala Tyr Gly Ile Asn Tyr Ala Asp Ser Val Lys Gly Arg Phe Thr Ile Ser Arg Asp Asn | |
| Ala Lys Asn Ser Leu Tyr Leu Gln Met Asn Ser Leu Arg Ala Glu Asp Thr Ala Val Tyr Tyr Cys Ala Arg Gly Ala Arg Ala Thr Gly Trp Phe Ala Tyr Trp Gly Gln Gly Thr Leu Val Thr Val Ser Ser | |
| **SEQ ID NO. 28** | **L0_15A7.5** |
| Asp Ile Gln Met Thr Gln Ser Pro Ser Ser Leu Ser Ala Ser Val Gly Asp Arg Val Thr Ile Thr Cys Arg Ala Ser Gln Asn Val Asp Thr His Val Ala Trp Phe Gln Gln Lys Pro Gly Lys Ala Pro Lys Ser Leu Ile Tyr Ser Ala Ser Tyr Leu Gln Ser Gly Val Pro Ser Arg Phe Ser Gly Ser Gly Ser Gly Thr Asp Phe Thr Leu Thr Ile Ser Ser Leu Gln Pro Glu Asp Phe Ala Thr Tyr Tyr Cys Gln Gln Tyr Asn Ser Tyr Pro Leu Thr Phe Gly Gly Gly Thr Lys Val Glu Ile Lys | |
| **SEQ ID NO. 29** | **CDR1 H1_15A7.5 IMGT** |
| Gly Phe Thr Phe Ser Asn Tyr Gly | |
| **SEQ ID NO. 30** | **CDR2 H1_15A7.5 IMGT** |
| Ile Ser Asn Leu Ala Tyr Gly Ile | |
| **SEQ ID NO. 31** | **CDR3 H1_15A7.5 IMGT** |
| Ala Arg Gly Ala Arg Ala Thr Gly Trp Phe Ala Tyr | |
| **SEQ ID NO. 32** | **CDR1 L1_15A7.5 IMGT** |
| Gln Asn Val Asp Thr His | |
| **SEQ ID NO. 33** | **CDR2 L1_15A7.5 IMGT** |
| Ser Ala Ser | |
| **SEQ ID NO. 34** | **CDR3 L1_15A7.5 IMGT** |
| Gln Gln Tyr Asn Ser Tyr Pro Leu Thr | |
| **SEQ ID NO. 35** | **CDR1 H1_15A7.5 Kabat** |
| Asn Tyr Gly Met Ala | |
| **SEQ ID NO. 36** | **CDR2 H1_15A7.5 Kabat** |
| Phe Ile Ser Asn Leu Ala Tyr Gly Ile Asn Tyr Ala Asp Thr Val Thr Gly | |
| **SEQ ID NO. 37** | **CDR3 H1_15A7.5 Kabat** |
| Gly Ala Arg Ala Thr Gly Trp Phe Ala Tyr | |
| **SEQ ID NO. 38** | **CDR1 L1_15A7.5 Kabat** |
| Lys Ala Ser Gln Asn Val Asp Thr His Val Ala | |
| **SEQ ID NO. 39** | **CDR2 L1_15A7.5 Kabat** |
| Ser Ala Ser Tyr Arg Tyr Ser | |
| **SEQ ID NO. 40** | **CDR3 L1_15A7.5 Kabat** |
| Gln Gln Tyr Asn Ser Tyr Pro Leu Thr | |
| **SEQ ID NO. 41** | **H1_15A7.5** |
| Glu Val Gln Leu Val Glu Ser Gly Gly Gly Leu Val Gln Pro Gly Gly Ser Leu | |
| Arg Leu Ser Cys Ala Ala Ser Gly Phe Thr Phe Ser Asn Tyr Gly Met Ala Trp Val Arg Gln Ala Pro Gly Lys Gly Leu Glu Trp Val Ser Phe Ile Ser Asn Leu Ala Tyr Gly Ile Asn Tyr Ala Asp Thr Val Thr Gly Arg Phe Thr Ile Ser Arg Asp Asn Ala Lys Asn Ser Leu Tyr Leu Gln Met Asn Ser Leu Arg Ala Glu Asp Thr Ala Val Tyr Tyr Cys Ala Arg Gly Ala Arg Ala Thr Gly Trp Phe Ala Tyr Trp Gly Gln Gly Thr Leu Val Thr Val Ser Ser | |
| **SEQ ID NO. 42** | **L1_15A7.5** |
| Asp Ile Gln Met Thr Gln Ser Pro Ser Ser Leu Ser Ala Ser Val Gly Asp Arg Val Thr Ile Thr Cys Cys Lys Ala Ser Gln Asn Val Asp Thr His Val Ala Trp Phe Gln Gln Lys Pro Gly Lys Ala Pro Lys Ser Leu Ile Tyr Ser Ala Ser Tyr Arg Tyr Ser Gly Val Pro Ser Arg Phe Ser Gly Ser Gly Ser Gly Thr Asp Phe Thr Leu Thr Ile Ser Ser Leu Gln Pro Glu Asp Phe Ala Thr Tyr Tyr Cys Gln Gln Tyr Asn Ser Tyr Pro Leu Thr Phe Gly Gly Gly Thr Lys Val Glu Ile Lys | |
| **SEQ ID NO. 43** | **CDR1 H2_15A7.5 IMGT** |
| Gly Phe Thr Phe Ser Asn Tyr Gly | |
| **SEQ ID NO. 44** | **CDR2 H2_15A7.5 IMGT** |
| Ile Ser Asn Leu Ala Tyr Gly Ile | |
| **SEQ ID NO. 45** | **CDR3 H2_15A7.5 IMGT** |
| Ala Arg Gly Ala Arg Ala Thr Gly Trp Phe Ala Tyr | |
| **SEQ ID NO. 46** | **CDR1 L2_15A7.5 IMGT** |
| Gln Asn Val Asp Thr His | |
| **SEQ ID NO. 47** | **CDR2 L2_15A7.5 IMGT** |
| Ser Ala Ser | |
| **SEQ ID NO. 48** | **CDR3 L2_15A7.5 IMGT** |
| Gln Gln Tyr Asn Ser Tyr Pro Leu Thr | |
| **SEQ ID NO. 49** | **CDR1 H2_15A7.5 Kabat** |
| Asn Tyr Gly Met Ala | |
| **SEQ ID NO. 50** | **CDR2 H2_15A7.5 Kabat** |
| Phe Ile Ser Asn Leu Ala Tyr Gly Ile Asn Tyr Ala Asp Thr Val Thr Gly | |
| **SEQ ID NO. 51** | **CDR3 H2_15A7.5 Kabat** |
| Gly Ala Arg Ala Thr Gly Trp Phe Ala Tyr | |
| **SEQ ID NO. 52** | **CDR1 L2_15A7.5 Kabat** |
| Lys Ala Ser Gln Asn Val Asp Thr His Val Ala | |
| **SEQ ID NO. 53** | **CDR2 L2_15A7.5 Kabat** |
| Ser Ala Ser Tyr Arg Tyr Ser | |
| **SEQ ID NO. 54** | **CDR3 L2_15A7.5 Kabat** |
| Gln Gln Tyr Asn Ser Tyr Pro Leu Thr | |
| **SEQ ID NO. 55** | **H2_15A7.5** |
| Glu Val Gln Leu Val Glu Ser Gly Gly Gly Leu Val Gln Pro Gly Gly Ser Leu Arg Leu Ser Cys Ala Ala Ser Gly Phe Thr Phe Ser Asn Tyr Gly Met Ala Trp Val Arg Gln Ala Pro Gly Lys Gly Leu Glu Trp Val Ala Phe Ile Ser Asn Leu Ala Tyr Gly Ile Asn Tyr Ala Asp Thr Val Thr Gly Arg Phe Thr Ile Ser Arg Asp Asn Ala Lys Asn Ser Leu Tyr Leu Gln Met Asn Ser Leu Arg Ala Glu Asp Thr Ala Val Tyr Tyr Cys Ala Arg Gly Ala Arg Ala Thr Gly Trp Phe Ala Tyr Trp Gly Gln Gly Thr Leu Val Thr Val Ser Ser | |
| **SEQ ID NO. 56** | **L2_15A7.5** |
| Asp Ile Gln Met Thr Gln Ser Pro Ser Ser Leu Ser Ala Ser Val Gly Asp Arg Val Thr Ile Thr Cys Lys Ala Ser Gln Asn Val Asp Thr His Val Ala Trp Tyr Gln Gln Lys Pro Gly Lys Ala Pro Lys Ala Leu Ile Tyr Ser Ala Ser Tyr Arg Tyr Ser Gly Val Pro Ser Arg Phe Ser Gly Ser Gly Ser Gly Thr Asp Phe Thr Leu Thr Ile Ser Ser Leu Gln Pro Glu Asp Phe Ala Thr Tyr Tyr Cys Gln Gln Tyr Asn Ser Tyr Pro Leu Thr Phe Gly Gly Gly Thr Lys Val Glu Ile Lys | |
| **SEQ ID NO. 57** | **CDR1 H3_15A7.5 IMGT** |
| Gly Phe Thr Phe Ser Asn Tyr Gly | |
| **SEQ ID NO. 58** | **CDR2 H3_15A7.5 IMGT** |
| Ile Ser Asn Leu Ala Tyr Gly Ile | |
| **SEQ ID NO. 59** | **CDR3 H3_15A7.5 IMGT** |
| Ala Arg Gly Ala Arg Ala Thr Gly Trp Phe Ala Tyr | |
| **SEQ ID NO. 60** | **CDR1 L3_15A7.5 IMGT** |
| Gln Asn Val Asp Thr His | |
| **SEQ ID NO. 61** | **CDR2 L3_15A7.5 IMGT** |
| Ser Ala Ser | |
| **SEQ ID NO. 62** | **CDR3 L3_15A7.5 IMGT** |
| Gln Gln Tyr Asn Ser Tyr Pro Leu Thr | |
| **SEQ ID NO. 63** | **CDR1 H3_15A7.5 Kabat** |
| Asn Tyr Gly Met Ala | |
| **SEQ ID NO. 64** | **CDR2 H3_15A7.5 Kabat** |
| Phe Ile Ser Asn Leu Ala Tyr Gly Ile Asn Tyr Ala Asp Thr Val Thr | |
| Gly | |
| **SEQ ID NO. 65** | **CDR3 H3_15A7.5 Kabat** |
| Gly Ala Arg Ala Thr Gly Trp Phe Ala Tyr | |
| **SEQ ID NO. 66** | **CDR1 L3_15A7.5 Kabat** |
| Lys Ala Ser Gln Asn Val Asp Thr His Val Ala | |
| **SEQ ID NO. 67** | **CDR2 L3_15A7.5 Kabat** |
| Ser Ala Ser Tyr Arg Tyr Ser | |
| **SEQ ID NO. 68** | **CDR3 L3_15A7.5 Kabat** |
| Gln Gln Tyr Asn Ser Tyr Pro Leu Thr | |
| **SEQ ID NO. 69** | **H3_15A7.5** |
| Glu Val Gln Leu Val Glu Ser Gly Gly Gly Leu Val Gln Pro Gly Gly Ser Leu Arg Leu Ser Cys Ala Ala Ser Gly Phe Thr Phe Ser Asn Tyr Gly Met Ala Trp Val Arg Gln Ala Pro Gly Lys Gly Pro Glu Trp Val Ala Phe Ile Ser Asn Leu Ala Tyr Gly Ile Asn Tyr Ala Asp Thr Val Thr Gly Arg Phe Thr Ile Ser Arg Asp Asn Ala Lys Asn Ser Leu Tyr Leu Gln Met Asn Ser Leu Arg Ala Glu Asp Thr Ala Val Tyr Tyr Cys Ala Arg Gly Ala Arg Ala Thr Gly Trp Phe Ala Tyr Trp Gly Gln Gly Thr Leu Val Thr Val Ser Ser | |
| **SEQ ID NO. 70** | **L3_15A7.5** |
| Asp Ile Gln Met Thr Gln Ser Pro Ser Ser Leu Ser Ala Ser Val Gly Asp Arg Val Thr Ile Thr Cys Lys Ala Ser Gln Asn Val Asp Thr His Val Ala Trp Tyr Tyr Gln Gln Lys Pro Gly Lys Ser Pro Lys Ala Leu Ile Tyr Ser Ala Ser Tyr Arg Tyr Ser Gly Val Pro Ser Arg Phe Ser Gly Ser Gly Ser Gly Thr Asp Phe Thr Leu Thr Ile Ser Ser Leu Gln Pro Glu Asp Phe Ala Thr Tyr Tyr Cys Gln Gln Tyr Asn Ser Tyr Pro Leu Thr Phe Gly Gly Gly Thr Lys Val Glu Ile Lys | |
| **SEQ ID NO. 71** | **L3_15A7.5_mod** |
| Asp Ile Gln Met Thr Gln Ser Pro Ser Ser Leu Ser Ala Ser Val Gly Asp Arg Val Thr Ile Thr Cys Lys Ala Ser Gln Asn Val Asp Thr His Val Ala Trp Tyr Tyr Gln Gln Lys Pro Gly Lys Ser Pro Lys Ala Leu Ile Tyr Ser Ala Ser Tyr Arg Tyr Ser Gly Val Pro Ser Arg Phe Ser Gly Ser Gly Ser Gly Thr Asp Phe Thr Leu Thr Ile Ser Ser Leu Gln Pro Glu Asp Phe Ala Thr Tyr Cys Gln Gln Tyr Asn Ser Tyr Pro LeuThr Phe Gly Gly Gly Thr Lys Val Glu Ile Lys | |
| **SEQ ID NO. 72** | **CDR1 VH_HA22** |
| Gly Phe Thr Phe Ser Ser Tyr Asn | |
| **SEQ ID NO. 73** | **CDR2 VH_HA22** |
| Ile Ser Ser Ser Ser Ser Thr Ile | |
| **SEQ ID NO. 74** | **CDR3 VH_HA22** |
| Ala Arg Ala Tyr Tyr Tyr Gly Met Asp Val | |
| **SEQ ID NO. 75** | **CDR1 VK_HA22** |
| Gln Gly Ile Ser Gly Trp | |
| **SEQ ID NO. 76** | **CDR2 VK_HA22** |
| Ala Ala Ser | |
| **SEQ ID NO. 77** | **CDR3 VK_HA22** |
| Gln Gln Ala Asn Ser Phe Pro Pro Thr | |
| **SEQ ID NO. 78** | **VH_HA22** |
| Glu Val Gln Leu Val Glu Ser Gly Gly Gly Leu Val Gln Pro Gly Gly Ser Leu Arg Leu Ser Cys Ala Ala Ser Gly Phe Thr Phe Ser Ser Tyr Asn Met Asn Trp Val Arg Gln Ala Pro Gly Lys Gly Leu Glu Trp Val Ser Tyr Ile Ser Ser Ser Ser Ser Thr Ile Tyr Tyr Ala Asp Ser Val Lys Gly Arg Phe Thr Ile Ser Arg Asp Asn Ala Lys Asn Ser Leu Ser Leu Gln Met Asn Ser Leu Arg Asp Glu Asp Thr Ala Val Tyr Tyr Cys Ala Arg Ala Tyr Tyr Tyr Gly Met Asp Val Trp Gly Gln Gly Thr Thr Val Thr Val Ser Ser | |
| **SEQ ID NO. 79** | **VK_HA22** |
| Asp Ile Gln Met Thr Gln Ser Pro Ser Ser Val Ser Ala Ser Val Gly Asp Arg Val Thr Ile Thr Cys Arg Ala Ser Gln Gly Ile Ser Gly Trp Leu Ala Trp Tyr Gln Gln Lys Pro Gly Lys Ala Pro Lys Phe Leu Ile Tyr Ala Ala Ser Thr Leu Gln Ser Gly Val Pro Ser Arg Phe Ser Gly Ser Gly Ser Gly Thr Asp Phe Thr Leu Thr Ile Ser Ser Leu Gln Pro Glu Asp Phe Ala Thr Tyr Tyr Cys Gln Gln Ala Asn Ser Phe Pro Pro Thr Phe Gly Gly Gly Thr Lys Val Glu Ile Lys | |
| **SEQ ID NO. 80** | **CDR1 VH_5A12.2** |
| Gly Phe Thr Phe Asn Ser Met Tyr | |
| **SEQ ID NO. 81** | **CDR2 VH_5A12.2** |
| Ile Tyr Ala Gly Thr Gly Gly Thr | |
| **SEQ ID NO. 82** | **CDR3 VH_5A12.2** |
| Ala Ile Arg Ser Gly Phe Val Pro Met Asp Tyr Trp Gly | |
| **SEQ ID NO. 83** | **VH_5A12.2** |
| Gln Ile Gln Leu Gln Gln Ser Gly Ala Glu Leu Val Lys Pro Gly Ala Ser Val Thr Leu Ser Cys Lys Thr Ser Gly Phe Thr Phe Asn Ser Met Tyr Ile Ser Trp Leu Lys Gln Lys Pro Gly Gln Ser Leu Glu Trp Ile Ala Trp Ile Tyr Ala Gly Thr Gly Gly Thr Arg Phe Asn Gln Lys Phe Thr Gly Lys Val Gln Leu Thr Val Asp Thr Ser Ser Ser Thr Ala Tyr Met Gln Phe Ser Ser Leu Thr Thr Asp Asp Ser Ala Ile Tyr Tyr Cys Ala Ile Arg Ser Gly Phe Val Pro Met Asp Tyr Trp Gly Gln Gly Thr Ser Val Thr Val Ser Ser | |
| **SEQ ID NO. 84** | **CDR1 VK_5A12.2** |
| Gln Ser Val Ser Asn Asp | |
| **SEQ ID NO. 85** | **CDR2 VK_5A12.2** |
| Tyr Ala Ser | |
| **SEQ ID NO. 86** | **CDR3 VK_5A12.2** |
| Gln Gln Asp Tyr Ser Ser | |
| **SEQ ID NO. 87** | **VK_5A12.2** |
| Ser Ile Val Met Thr Gln Thr Pro Lys Phe Leu Leu Val Ser Ala Gly Asp Arg Val | |
| Thr Ile Thr Cys Lys Ala Ser Gln Ser Val Ser Asn Asp Val Ala Trp Tyr Gln Gln Lys Pro Gly Gln Ser Pro Lys Leu Leu Ile Tyr Tyr Ala Ser Asn Arg Tyr Thr Gly Val Pro Asp Arg Phe Thr Gly Ser Gly Tyr Gly Thr Asp Phe Thr Phe Thr Ile Ser Thr Val Gln Ala Glu Asp Leu Ala Val Tyr Phe Cys Gln Gln Asp Tyr Ser Ser Pro Trp Thr Phe Gly Gly Gly Thr Lys Leu Glu Ile Lys | |
| **SEQ ID NO. 88** | **CDR1 VH_9A2.7** |
| Gly Tyr Asn Phe Thr Thr Phe Trp | |
| **SEQ ID NO. 89** | **CDR2 VH_9A2.7** |
| Ile Tyr Pro Ser Asp Ser Tyr Ala | |
| **SEQ ID NO. 90** | **CDR3 VH_9A2.7** |
| Thr Arg Pro Ser Tyr Phe Gly Arg Asn Ser Phe Ala Tyr | |
| **SEQ ID NO. 91** | **VH_9A2.7** |
| Gln Val Gln Leu Gln Gln Pro Gly Ala Glu Leu Val Arg Pro Gly Ala Ser Val Lys Leu Ser Cys Lys Ala Ser Gly Tyr Asn Phe Thr Thr Phe Trp Ile Asn Trp Val Lys Gln Arg Pro Gly Gln Gly Leu Glu Trp Ile Gly Asn Ile Tyr Pro Ser Asp Ser Tyr Ala Asn Tyr Asn Gln Lys Phe Lys Asp Lys Ala Thr Leu Thr Val Asp Lys Ser Ser Thr Thr Ala Tyr Met Gln Leu Ser Ser Pro Thr Ser Glu Asp Ser Ala Val Tyr Tyr Cys Thr Arg Pro Ser Tyr Phe Gly Arg Asn Ser Phe Ala Tyr Trp Gly Gln Gly Thr Leu Val Thr Val Ser Ala | |
| **SEQ ID NO. 92** | **CDR1 VK_9A2.7** |
| Gln Ser Leu Leu Tyr Ser Val Asn His Lys Asn Tyr | |
| **SEQ ID NO. 93** | **CDR2 VK_9A2.7** |
| Trp Ala Ser | |
| **SEQ ID NO. 94** | **CDR3 VK_9A2.7** |
| His Gln Tyr Tyr Thr Tyr Pro Leu Thr | |
| **SEQ ID NO. 95** | **VK_9A2.7** |
| Asp Ile Val Met Thr Gln Ser Pro Ser Ser Leu Val Val Ser Val Gly Glu Lys Val Thr Met Ser Cys Lys Ser Ser Gln Ser Leu Leu Tyr Ser Val Asn His Lys Asn Tyr Leu Ala Trp Tyr Gln Gln Lys Pro Gly Gln Ser Pro Lys Leu Leu Ile Tyr Trp Ala Ser Thr Arg Glu Ser Gly Val Pro Asp Arg Phe Thr Gly Ser Gly Ser Gly Thr Asp Phe Ser Leu Thr Ile Ser Ser Val Lys Ala Glu Asp Leu Ala Val Tyr Tyr Cys His Gln Tyr Tyr Thr Tyr Pro Leu Thr Phe Gly Ala Gly Thr Lys Leu Glu Leu Lys | |
| **SEQ ID NO. 96** | **CDR1 VH_b3A1.4** |
| Gly Asp Ser Ile Thr Ser Gly Tyr | |
| **SEQ ID NO. 97** | **CDR2 VH_3A1.4** |
| Ile Ser Asn Ser Gly Ile Thr | |
| **SEQ ID NO. 98** | **CDR3 VH_3A1.4** |
| Thr Arg Phe Gly Ser Thr Met Ile Leu Tyr Tyr Thr Met Asp Tyr | |
| **SEQ ID NO. 99** | **VH_3A1.4** |
| Asp Val Gln Leu Gln Glu Ser Gly Pro Ser Leu Val Lys Pro Ser Gln Thr Leu Ser Leu Thr Cys Ser Val Thr Gly Asp Ser Ile Thr Ser Gly Tyr Trp Asn Trp Ile Arg Lys Phe Pro Gly Asn Lys Leu Glu Tyr Met Gly Tyr Ile Ser Asn Ser Gly Ile Thr Tyr Tyr Asn Pro Ser Leu Lys Ser Arg Ile Ser Ile Thr Arg Asp Thr Ser Lys Asn Gln Tyr Phe Leu Gln Leu Asn Ser Val Thr Ala Glu Asp Thr Ala Thr Tyr Tyr Cys Thr Arg Phe Gly Ser Thr Met Ile Leu Tyr Tyr Thr Met Asp Tyr Trp Gly Gln Gly Thr Ser Val Thr Val Ser Ser | |
| **SEQ ID NO. 100** | **CDR1 VK_3A1.4** |
| Gln Ser Ile Ser Asp Tyr | |
| **SEQ ID NO. 101** | **CDR2 VK_3A1.4** |
| Tyr Ala Ser | |
| **SEQ ID NO. 102** | **CDR3 VK_3A1.4** |
| Gln Asn Gly His Ser Phe Pro Leu Thr | |
| **SEQ ID NO. 103** | **VK_3A1.4** |
| Asp Ile Val Met Thr Gln Ser Pro Val Thr Leu Ser Val Thr Pro Gly Asp Arg Val Ser Leu Ser Cys Arg Ala Ser Gln Ser Ile Ser Asp Tyr Leu His Trp Tyr Gln Gln Lys Ser Gln Glu Ser Pro Arg Leu Leu Ile Lys Tyr Ala Ser Lys Ser Ile Ser Gly Ile Pro Ser Arg Phe Ser Gly Ser Gly Ser Gly Ser Asn Phe Thr Leu Ser Ile Asn Ser Val Glu Pro Glu Asp Val Gly Val Tyr Tyr Cys Gln Asn Gly His Ser Phe Pro Leu Thr Phe Gly Ala Gly Thr Lys Leu Glu Leu Lys | |
| **SEQ ID NO. 104** | **CDR1 VH_8F06** |
| Gly Tyr Thr Phe Thr Ser Tyr Val | |
| **SEQ ID NO. 105** | **CDR2 VH_8F06** |
| Ile Asn Pro Tyr Asn Asp Gly Thr | |
| **SEQ ID NO. 106** | **CDR3 VH_8F06** |
| Val Thr Leu Phe Ala Tyr | |
| **SEQ ID NO. 107** | **VH_8F06** |
| Glu Val Gln Leu Gln Gln Ser Gly Pro Glu Leu Val Lys Pro Gly Ala Ser Val Lys Met Ser Cys Lys Ala Ser Gly Tyr Thr Phe Thr Ser Tyr Val Met His Trp Val Lys Gln Lys Pro Gly Gln Gly Leu Glu Trp Ile Gly Tyr Ile Asn Pro Tyr Asn Asp Gly Thr Lys Tyr Asn Glu Lys Phe Lys Gly Lys Ala Thr Leu Thr Ser Asp Lys Ser Ser Ser Thr Ala Tyr Met Glu Leu Ser Ser Leu Thr Ser Glu Asp Ser Ala Val Tyr Tyr Cys Val Thr Leu Phe Ala Tyr Trp Gly Gln Gly Thr Leu Val Thr Val Ser Ala | |
| **SEQ ID NO. 108** | **CDR1 VK_8F06** |
| Ser Ser Ile Ser Tyr | |
| **SEQ ID NO. 109** | **CDR2 VK_8F06** |
| Asp Thr Ser | |
| **SEQ ID NO. 110** | **CDR3 VK_8F06** |
| His Gln Arg Ser Ser Tyr Pro Phe Thr | |
| **SEQ ID NO. 111** | **VK_8F06** |
| Gln Ile Val Leu Thr Gln Ser Pro Ala Ile Met Ser Ala Ser Pro Gly Glu Lys Val Thr Met Thr Cys Ser Ala Ser Ser Ser Ile Ser Tyr Met His Trp Tyr Gln Gln Lys Pro Gly Thr Ser Pro Lys Arg Trp Ile Tyr Asp Thr Ser Lys Leu Ala Ser Gly Val Pro Ala Arg Phe Ser Gly Ser Gly Ser Gly Thr Ser Tyr Ser Leu Thr Ile Ser Ser Met Glu Ala Glu Asp Ala Ala Thr Tyr Tyr Cys His Gln Arg Ser Ser Tyr Pro Phe Thr Phe Gly Ser Gly Thr Lys Leu Glu Ile Lys | |
| **SEQ ID NO. 112** | **Nectin-4** |
| Met Pro Leu Ser Leu Gly Ala Glu Met Trp Gly Pro Glu Ala Trp Leu Leu Leu Leu Leu Leu Leu Ala Ser Phe Thr Gly Arg Cys Pro Ala Gly Glu Leu Glu Thr Ser Asp Val Val Thr Val Val Leu Gly Gln Asp Ala Lys Leu Pro Cys Phe Tyr Arg Gly Asp Ser Gly Glu Gln Val Gly Gln Val Ala Trp Ala Arg Val Asp Ala Gly Glu Gly Ala Gln Glu Leu Ala Leu Leu His Ser Lys Tyr Gly Leu His Val Ser Pro Ala Tyr Glu Gly Arg Val Glu Gln Pro Pro Pro Pro Arg Asn Pro Leu Asp Gly Ser Val Leu Leu Arg Asn Ala Val Gln Ala Asp Glu Gly Glu Tyr Glu Cys Arg Val Ser Thr Phe Pro Ala Gly Ser Phe Gln Ala Arg Leu Arg Leu Arg Val Leu Val Pro Pro Leu Pro Ser Leu Asn Pro Gly Pro Ala Leu Glu Glu Gly Gln Gly Leu Thr Leu Ala Ala Ser Cys Thr Ala Glu Gly Ser Pro Ala Pro Ser Val Thr Trp Asp Thr Glu Val Lys Gly Thr Thr Ser Ser Arg Ser Phe Lys His Ser Arg Ser Ala Ala Val Thr Ser Glu Phe His Leu Val Pro Ser Arg Ser Met Asn Gly Gln Pro Leu Thr Cys Val Val Ser His Pro Gly Leu Leu Gln Asp Gln Arg Ile Thr His Ile Leu His Val Ser Phe Leu Ala Glu Ala Ser Val Arg Gly Leu Glu Asp Gln Asn Leu Trp His Ile Gly Arg Glu Gly Ala Met Leu Lys Cys Leu Ser Glu Gly Gln Pro Pro Pro Ser Tyr Asn Trp Thr Arg Leu Asp Gly Pro Leu Pro Ser Gly Val Arg Val Asp Gly Asp Thr Leu Gly Phe Pro Pro Leu Thr Thr Glu His Ser Gly Ile Tyr Val Cys His Val Ser Asn Glu Phe Ser Ser Arg Asp Ser Gln Val Thr Val Asp Val Leu Asp Pro Gln Glu Asp Ser Gly Lys Gln Val Asp Leu Val Ser Ala Ser Val Val Val Val Gly Val Ile Ala Ala Leu Leu Phe Cys Leu Leu Val Val Val Val Val Leu Met Ser Arg Tyr His Arg Arg Lys Ala Gln Gln Met Thr Gln Lys Tyr Glu Glu Glu Leu Thr Leu Thr Arg Glu Asn Ser Ile Arg Arg Leu His Ser His His Thr Asp Pro Arg Ser Gln Pro Glu Glu Ser Val Gly Leu Arg Ala Glu Gly His Pro Asp Ser Leu Lys Asp Asn Ser Ser Cys Ser Val Met Ser Glu Glu Pro Glu Gly Arg Ser Tyr Ser Thr Leu Thr Thr Val Arg Glu Ile Glu Thr Gln Thr Glu Leu Leu Ser Pro Gly Ser Gly Arg Ala Glu Glu Glu Glu Asp Gln Asp Glu Gly Ile Lys Gln Ala Met Asn His Phe Val Gln Glu Asn Gly Thr Leu Arg Ala Lys Pro Thr Gly Asn Gly Ile Tyr Ile Asn Gly Arg Gly His Leu Val | |

## Claims

1. An antibody-drug conjugate according to Formula (I) 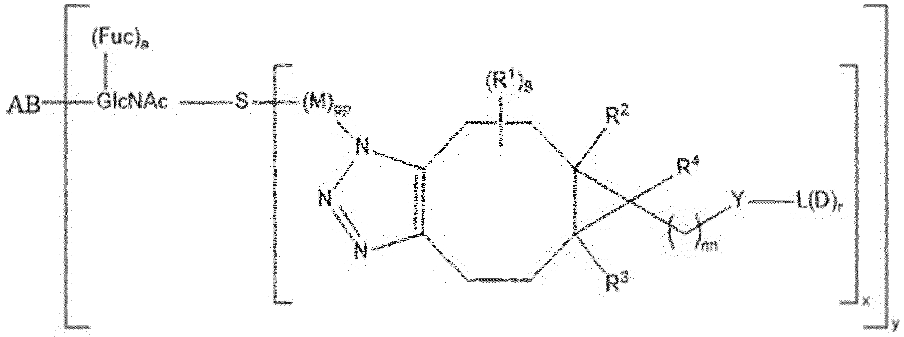 wherein
AB is an anti-Nectin-4 antibody or functional fragment thereof,
Fuc is fucose;
GlcNAc is N-acetylglucosamine;
S is a sugar or sugar derivative;
M is -N(H)C(O)CH₂-, -N(H)C(O)CF₂-, -CH₂-, -CF₂- or a 1,4-phenylene containing 0-4 fluorine substituents, preferably 2 fluorine substituents which are preferably positioned on C2 and C6 or on C3 and C5 of the phenylene;
R¹ is independently selected from the group consisting of hydrogen, halogen, -OR⁸, -NO₂, -CN, S(O)₂R⁸, C₁-C₂₄ alkyl groups, C₆-C₂₄ (hetero)aryl groups, C₇-C₂₄ alkyl(hetero)aryl groups and C₇-C₂₄ (hetero)arylalkyl groups, preferably hydrogen, and wherein the alkyl groups, (hetero)aryl groups, alkyl(hetero)aryl groups and (hetero)arylalkyl groups are optionally substituted, wherein two substituents R⁸ may be linked together to form an annelated cycloalkyl or an annelated (hetero)arene substituent, and wherein R⁸ is independently selected from the group consisting of hydrogen, halogen, C₁-C₂₄ alkyl groups, C₆-C₂₄ (hetero)aryl groups, C₇-C₂₄ alkyl(hetero)aryl groups and C₇-C₂₄ (hetero)arylalkyl groups;
R² and are R³ are independently selected from the group consisting of hydrogen, halogen, C₁-C₂₄ alkyl groups, C₆-C₂₄ (hetero)aryl groups, C₇-C₂₄ alkyl(hetero)aryl groups and C₇-C₂₄ (hetero)arylalkyl groups, preferably hydrogen;
R⁴ is selected from the group consisting of hydrogen, halogen, C₁-C₂₄ alkyl groups, C₆-C₂₄ (hetero)aryl groups, C₇-C₂₄ alkyl(hetero)aryl groups and C₇-C₂₄ (hetero)arylalkyl groups, preferably hydrogen, the alkyl groups optionally being interrupted by one of more hetero-atoms selected from the group consisting of O, N and S, wherein the alkyl groups, (hetero)aryl groups, alkyl(hetero)aryl groups and (hetero)arylalkyl groups are independently optionally substituted;
Y is O, S or NR⁷, wherein R⁷ is independently selected from the group consisting of hydrogen, halogen, -OR⁸, -NO₂, -CN, S(O)₂R⁸, C₁-C₂₄ alkyl groups, C₆-C₂₄ (hetero)aryl groups, C₇-C₂₄ alkyl(hetero)aryl groups and C₇-C₂₄ (hetero)arylalkyl groups and wherein the alkyl groups, (hetero)aryl groups, alkyl(hetero)aryl groups and (hetero)arylalkyl groups are optionally substituted, wherein two substituents R⁸ may be linked together to form an annelated cycloalkyl or an annelated (hetero)arene substituent and wherein R⁸ is independently selected from the group consisting of hydrogen, halogen, C₁-C₂₄ alkyl groups, C₆-C₂₄ (hetero)aryl groups, C₇-C₂₄ alkyl(hetero)aryl groups and C₇-C₂₄ (hetero)arylalkyl groups;
L is a linking group, preferably a sulfamide based linking group,
a is 0 or 1;
x is 1 or 2, preferably 1;
y is 1, 2, 3 or 4, preferably 1,
r is 1, 2, 3, 4, 5, 6, 7, 8, 9, 10, 11, 12, 13, 14, 15, 16, 17, 18, 19 or 20;
nn is 1, 2, 3, 4, 5, 6, 7, 8, 9 or 10, preferably 1 or 2, in particular 1;
pp is 0 or 1;
D is is a topoisomerase I inhibitor, in particular exatecan.

2. The antibody-drug conjugate of claim 1, wherein L comprises a group according to Formula (II) 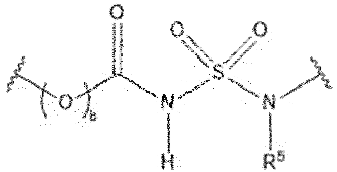 wherein b is 0 or 1;
R⁵ is selected from the group consisting of hydrogen, C₁-C₂₄ alkyl groups, C3-C24 cycloalkyl groups, C₂-C₂₄ (hetero)aryl groups, C₃-C₂₄ alkyl(hetero)aryl groups and C₃-C₂₄(hetero)arylalkyl groups, preferably hydrogen,
the C₁-C₂₄ alkyl groups, C₃-C₂₄ cycloalkyl groups, C₂-C₂₄ (hetero)aryl groups, C₃-C₂₄ alkyl(hetero)aryl groups and C₃-C₂₄(hetero)arylalkyl groups optionally substituted and/or optionally interrupted by one or more heteroatoms selected from O, S or NR⁶, wherein R⁶ is hydrogen or C₁-C₄ alkyl, preferably hydrogen; wherein D is optionally connected to N via a spacer moiety.

3. The antibody-drug conjugate of claim 2,
wherein the spacer moiety is represented by Formula (III)
Q-(L¹)ₙ-(CO)-(L²)ₒ-(L³)ₚ-(L⁴)_{q}- ,
wherein
Q is -(W)ₖ-(A)_{d}-(B)ₑ-(A¹)_{f}- , wherein
W is -OC(O)-, -C(O)O-, -C(O)NH-, -NHC(O)-, -OC(O)NH-,-NHC(O)O-, -C(O)(CH₂)ₘC(O)-, -C(O)(CH₂)ₘC(O)NH-, or-(4-Ph)CH₂NHC(O)(CH₂)ₘC(O)NH-, wherein m is an integer in the range 0-10;
A is of Formula (II) as defined above;
A¹ is of Formula (II) as defined above, with R⁵ being preferably (L¹)ₙ-(CO)-(L²)ₒ-(L³)ₚ-(L⁴)_{q}-,
B is a -CH₂-CH₂-O- or a -O-CH₂-CH₂- moiety, or (B)_{g} is a-(CH₂-CH₂-O)_{g}-CH₂CH₂- moiety, wherein g is an integer in the range 1 - 10;
k is 0 or 1, with the proviso that if k is 1 then d is 0;
d is 0 or 1;
e is an integer in the range 1 - 10;
f is 0 or 1;
L¹ is a -CH₂-CH₂-O-, -O-CH₂-CH₂-, or -(CH₂-CH₂-O)ₙ₁-CH₂-CH₂-moiety, wherein n1 is an independently selected integer in the range of 1-10,
L² is a peptide spacer, preferably a dipeptide wherein L² is represented by general structure (IV): 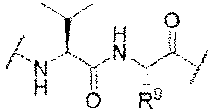 wherein R⁹ is hydrogen, CH₃ or CH₂CH₂CH₂NHC(O)NH₂;
L³ is self-immolative spacer, preferably a para-aminobenzyloxycarbonyl (PABC) derivative, in particular a para-aminobenzyloxycarbonyl (PABC) derivative according to structure (V) 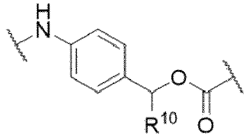
wherein R¹⁰ is hydrogen, R¹¹ or C(O)R¹¹ ,
wherein R¹¹ is C₁-C₂₄ (hetero)alkyl groups, C₃-C₁₀ (hetero)cycloalkyl groups, C₂-C₁₀ (hetero)aryl groups, C₃-C₁₀ alkyl(hetero)aryl groups and C₃-C₁₀ (hetero)arylalkyl groups, which are optionally substituted and optionally interrupted by one or more heteroatoms selected from O, S and NR¹² wherein R¹² is independently selected from the group consisting of hydrogen and C₁-C₄ alkyl groups, wherein R¹⁰ preferably being hydrogen or C(O)R¹¹ wherein R¹¹ is preferably 4-methyl-piperazine or morpholine, most preferably wherein R¹⁰ being hydrogen;
L⁴ is an aminoalkanoic acid spacer according to the structure -N-(Cₓ-alkylene)-C(O)-, wherein x is an integer in the range 1-10, or L⁴ is an ethyleneglycol spacer according to the structure -N-(CH₂-CH₂-O)ₓ₁-(CH₂)ₓ₂-C(O)-, wherein x1 is an integer in the range 1-10 and x2 is an integer in the range 1-3; and
n, o, p, q are independently selected from 0 or 1.

4. The antibody-drug conjugate of any of claims 1-3, wherein R¹, R², R³ and R⁴ are hydrogen.

5. The antibody-drug conjugate of any one of claims 1 - 4,
comprising an comprising an antibody, which specifically binds to Nectin-4 expressed by tumors with a higher affinity in comparison to Nectin-4 expressed by human differentiated keratinocytes, or an antigen-binding fragment thereof.

6. The antibody-drug conjugate of any one of claims 1-5,
wherein the Nectin-4-binding agent, particularly an antibody, specifically binds to a discontinuous epitope on Nectin-4 consisting of amino acids D57, S58, E60, P133, G135, F137, Q138, and R140 and optionally one or more of G56, G59, E126, R128, P133, and A134 of SEQ ID NO: 112 as determined using Deep Mutational Scanning (DMS).

7. The antibody-drug conjugate of any one of claims 1-6 wherein the Nectin-4-binding agent is an antibody or antigen-binding fragment thereof comprising:
(a)
(i) a VH region comprising the CDR-H1 of SEQ ID NO:1, the CDR-H2 of SEQ ID NO:2, and the CDR-H3 of SEQ ID NO:3, and
(ii) a VL region comprising the CDR-L1 of SEQ ID NO:4, the CDR-L2 of the SEQ ID NO:5, and the CDR-L3 of the SEQ ID NO:6,
or
(b)
(i) a VH region comprising the CDR-H1 of SEQ ID NO:7, the CDR-H2 of SEQ ID NO:8, and the CDR-H3 of SEQ ID NO:9, and
(ii) a VL region comprising the CDR-L1 of SEQ ID NO:10, the CDR-L2 of the SEQ ID NO:11, and the CDR-L3 of the SEQ ID NO:12, or
(c)
(i) a VH region comprising the CDR-H1 of SEQ ID NO:88, the CDR-H2 of SEQ ID NO:89, and the CDR-H3 of SEQ ID NO:90, and
(ii)a VL region comprising the CDR-L1 of SEQ ID NO:92, the CDR-L2 of the SEQ ID NO:93, and the CDR-L3 of the SEQ ID NO:94, or
(d)
(i) a VH region comprising the CDR-H1 of SEQ ID NO:96, the CDR-H2 of SEQ ID NO:97, and the CDR-H3 of SEQ ID NO:98, and
(ii) a VL region comprising the CDR-L1 of SEQ ID NO:100, the CDR-L2 of the SEQ ID NO:101, and the CDR-L3 of the SEQ ID NO:102,
or
(e)
(i) a VH region comprising the CDR-H1 of SEQ ID NO:104, the CDR-H2 of SEQ ID NO:105, and the CDR-H3 of SEQ ID NO:106, and
(ii) a VL region comprising the CDR-L1 of SEQ ID NO:108, the CDR-L2 of the SEQ ID NO:109, and the CDR-L3 of the SEQ ID NO:110.

8. The antibody-drug conjugate of any one of claims 1-7 wherein the Nectin-4-binding agent is an antibody or antigen-binding fragment thereof comprising:
(a)
(i) a VH region comprising an amino acid sequence according to SEQ ID NO:13 or an amino acid sequence having an identity thereof of at least 85%, at least 90%, at least 95% or at least 99%,
and
(ii) a VL region, particularly a VL region comprising an amino acid sequence according to SEQ ID NO:14 or an amino sequence having an identity thereof of at least 85%, at least 90%, at least 95% or at least 99%,
or
(b)
(i) a VH region comprising an amino acid sequence according to SEQ ID NO:91 or an amino acid sequence having an identity thereof of at least 85%, at least 90%, at least 95% or at least 99%,
and
(ii) a VL region, particularly a VL region comprising an amino acid sequence according to SEQ ID NO:95 or an amino sequence having an identity thereof of at least 85%, at least 90%, at least 95% or at least 99%,
or
(c)
(i) a VH region comprising an amino acid sequence according to SEQ ID NO:99 or an amino acid sequence having an identity thereof of at least 85%, at least 90%, at least 95% or at least 99%,
and
(ii) a VL region, particularly a VL region comprising an amino acid sequence according to SEQ ID NO:103 or an amino sequence having an identity thereof of at least 85%, at least 90%, at least 95% or at least 99%,
or
(d)
(i) a VH region comprising an amino acid sequence according to SEQ ID NO:107 or an amino acid sequence having an identity thereof of at least 85%, at least 90%, at least 95% or at least 99%,
and
(ii) a VL region, particularly a VL region comprising an amino acid sequence according to SEQ ID NO:111 or an amino sequence having an identity thereof of at least 85%, at least 90%, at least 95% or at least 99%.

9. The antibody-drug conjugate of any one of claims 1-8 wherein the Nectin-4-binding agent is an antibody or antigen-binding fragment thereof comprising:
(a) a variable heavy chain (VH) region comprising complementarity-determining regions (CDRs) CDR-H1, CDR-H2 and CDR-H3, wherein
(i) the CDR-H1 comprises an amino acid sequence according to SEQ ID NO:21, 35, 49 or 63,
(ii) the CDR-H2 comprises an amino acid sequence according to SEQ ID NO:22, 36, 50 or 64
(iii) the CDR-H3 comprises an amino acid sequence according to SEQ ID NO:23, 37, 51 or 65,
and
(b) a variable light chain (VL) region comprising complementarity-determining regions (CDRs) CDR-L1, CDR-L2 and CDR-L3, wherein
(i) the CDR-L1 comprises an amino acid sequence according to SEQ ID NO:24, 38, 52 or 66,
(ii) the CDR-L2 comprises an amino acid sequence according to SEQ ID NO:25, 39, 53 or 67,
(iii) the CDR-L3 comprises an amino acid sequence according to SEQ ID NO:26, 40, 54 or 68.

10. The antibody-drug conjugate of any one of claims 1-9 wherein the topoisomerase I inhibitor is a camptothecin or an analog thereof, particularly exatecan or deruxtecan, and more particularly exatecan.

11. A pharmaceutical composition comprising an active agent, which is an antibody-drug conjugate according to any of claims 1-10 and a pharmaceutically acceptable carrier and/or excipient.

12. Use of an antibody-drug conjugate of any one of claims 1-10 or a pharmaceutical composition of claim 11 in medicine, particularly in human medicine.

13. Antibody-drug conjugate of any one of claims 1-10 or a pharmaceutical composition of claim 11 for use in a method for the prevention and/or treatment of a Nectin-4 associated disorder, particularly for the prevention and/or the treatment of a Nectin-4 positive cancer, particularly for the prevention and/or the treatment of a cancer selected from bladder, urothelial, endometrial, cervical, colorectal, liver, thyroid, breast, pancreatic, lung, ovarian, head and neck and/or esophagus cancer.

14. Antibody-drug conjugate of any one of claims 1-10 or a pharmaceutical composition of claim 11 for use in a method for the prevention and/or treatment of a Nectin-4 associated disorder in a subject, wherein the subject is **characterized in that** the disorder is resistant against administration of a conjugate comprising a Nectin-4-binding agent and an auristatin.

15. The antibody-drug conjugate for use according to claim 14 wherein the disorder is resistant against administration of Enfortumab vedotin.
